# EUROPEAN PATENT APPLICATION

(11) **EP 4 483 966 A2**
(43) Date of publication of application: **01.01.2025**
(21) Application number: 24211564.0
(22) Date of filing: 02.02.2021
(51) Int. Cl.: A61P 43/00

(54) **METHODS AND COMPOSITIONS FOR TREATMENT OF DISEASES**

(30) Priority: 03.02.2020 US 202062969404 P; 26.03.2020 US 202063000265 P
(62) Divisional of application: 21750017.2
(71) Applicant: Tranexamic Technologies, LLC, Dallas, TX 75209 (US)
(72) Inventor: STEWART, Paul, Dallas, 75209-3522 (US); MURDOCK, Frank, Dallas, 75209-3522 (US)
(74) Representative: Whitfield, Gillian Janette

(57) **Abstract**

In an embodiment, the present disclosure relates to a method of ameliorating or preventing a disease or disease condition. Generally, the method includes administering a composition having a synthetic lysine analog, derivative, or mimetic. In some embodiments, the synthetic lysine analog, derivative, or mimetic interacts with cells associated with the disease or disease condition to inhibit or prevent replication, survival, or formation of the cells. In another embodiment, the present disclosure relates to a composition having a synthetic lysine analog, derivative, or mimetic for ameliorating or preventing a disease or disease condition. In a further embodiment, the present disclosure is related to a method and composition having a synthetic lysine analog, derivative, or mimetic for ameliorating aging. In an additional embodiment, the present disclosure is related to a method and composition having a synthetic lysine analog, derivative, or mimetic for treatment and prevention of cancer.

## Description

### CROSS-REFERENCE TO RELATED APPLICATIONS

This patent application claims priority from, and incorporates by reference the entire disclosure of, U.S. Provisional Patent Application No. 62/969,404 filed on February 3, 2020 and U.S. Provisional Patent Application No. 63/000,265 filed on March 26, 2020.

### TECHNICAL FIELD

The present disclosure relates generally to treatment of diseases and more particularly, but not by way of limitation, to methods and compositions for treatment of diseases.

### BACKGROUND

This section provides background information to facilitate a better understanding of the various aspects of the disclosure. It should be understood that the statements in this section of this document are to be read in this light, and not as admissions of prior art.

Diseases are a particular abnormal condition, generally not due to immediate external injury, that negatively affects the function and/or structure of an organism. In humans, diseases are often known to be medical conditions that have associated signs and symptoms and can be caused by external and/or internal factors such as, but not limited to, pathogens or internal dysfunctions. For example, internal dysfunctions of the immune system can inflict a variety of different diseases, including various forms of immunodeficiency, hypersensitivity, allergies, autoimmune disorders, and the like. There are various types of diseases that can include, without limitation, infectious diseases, deficiency diseases, hereditary diseases, including both genetic hereditary diseases and non-genetic hereditary diseases, physiological diseases, viral diseases, and the like. Additionally, diseases can be communicable or non-communicable diseases.

Several environmental factors, for example, diet and activity levels, along with various genetic factors, play a role in determining the susceptibility of obtaining different diseases, such as cardiovascular or liver diseases and different types of cancers. Additionally, aging causes a gradual decline in normal physiological functions and represents an additional risk factor for contracting several diseases.

A particular disease, cancer (or malignancy), is an abnormal growth of cells. There are more than 100 types of cancer, including, but not limited to, colon cancer, blood cancers (*e.g.,* lymphoma or leukemia), breast cancer, prostate cancer, stomach cancer, lung cancer, kidney cancer, pancreas cancer, skin cancer, and many others. Symptoms vary depending on the type, and current cancer treatments include chemotherapy, radiation, surgery, or other various therapeutics and combinations thereof.

Cancer has caused millions of deaths, and a recent study indicated that approximately 14.1 million new cancer cases occur each year, not including skin cancer other than melanoma. Typically, the most common types of cancer in males are lung cancer, prostate cancer, colorectal cancer, and stomach cancer. While in females, the most common types of cancer are breast cancer, colorectal cancer, lung cancer, and cervical cancer. If skin cancer, other than melanoma, were included in total new cancer cases each year, cancer cases would account for around 40% of all medical cases. In children, acute lymphoblastic leukemia and brain tumors are most common. Another recent study indicated that in a previous year about 165,000 children were diagnosed with cancer, each less than 15 years of age. The risk of cancer increases significantly with age, and many cancers occur more commonly in developed countries. Rates are increasing as more people live to an older age and as lifestyle changes occur in developing worlds.

Survival rates vary by cancer type and by the stage at which it is diagnosed. The survival rates range from majority survival to complete mortality five years after diagnosis. Once cancer has metastasized, prognosis normally becomes worse. Approximately one-half of patients receiving treatment for invasive cancer die from that cancer or its treatment (or treatment-related illness). Survival is worse in developing worlds, partly because the types of cancer that are most common there are harder to treat than those associated with developed countries. Those who survive cancer are shown to develop a second primary cancer at about twice the rate of those who have never been diagnosed. The increased risk is believed to be due to the random chance of developing any cancer, the likelihood of surviving the first cancer, the same risk factors that produced the first cancer, and unwanted side effects of treating the first cancer (particularly radiation therapy).

Additionally, in general, studies have shown that the accepted blood concentration of tranexamic acid required to inhibit formation of plasmin and provide antifibrinolysis is approximately 10-20 pg/ml (*i.e.,* approximately intravenous doses of 10-20 mg/kg). Studies have further indicated that intravenous doses of 80-100 mg/kg and above are unnecessary for antifibrinolysis, and furthermore, are considered to create a risk of seizure in situations utilizing these doses, for example, during heart surgery. However, contraindicated to these studies, one aspect described herein demonstrates lysine analogs, such as tranexamic acid, inhibit protein-protein interactions involved in various diseases (*e.g.,* cancer) at a concentration much higher than amounts previously studied and considered potentially too close to cytotoxic doses. For example, as demonstrated herein in further detail below, 350 mg/kg was utilized in mice studies, and in particular *in vitro* testing, the concentration of tranexamic acid required was around 2%, or about 2 mg/ml (100-200 times the 10-20 µg /ml needed for antifibrinolysis).

In view of the aforementioned, methods and compositions to prevent disease development and to inhibit survival and proliferation of diseased cells is highly desirable. As such, the present disclosure generally seeks to address the aforementioned with various methods and compositions for ameliorating diseases and disease conditions.

### SUMMARY OF THE INVENTION

This summary is provided to introduce a selection of concepts that are further described below in the Detailed Description. This summary is not intended to identify key or essential features of the claimed subject matter, nor is it to be used as an aid in limiting the scope of the claimed subject matter.

In an embodiment, the present disclosure relates to a method of ameliorating or preventing a disease or disease condition. In some embodiments, the method includes administering a composition having a synthetic lysine analog, derivative, or mimetic. In some embodiments, the synthetic lysine analog, derivative, or mimetic interacts with cells associated with the disease or disease condition to inhibit or prevent replication, survival, or formation of the cells.

In another embodiment, the present disclosure relates to a composition for ameliorating or preventing a disease or disease condition. In some embodiments, the composition includes a synthetic lysine analog, derivative, or mimetic. In some embodiments, the synthetic lysine analog, derivative, or mimetic interacts with cells associated with the disease or disease condition to inhibit or prevent replication, survival, or formation of the cells.

In an additional embodiment, the present disclosure relates to a method of ameliorating aging or impacting the aging process to a patient subjected to environmental factors effecting the aging process. In some embodiments, the method includes administering a composition having a synthetic lysine analog, derivative, or mimetic. In some embodiments, the synthetic lysine analog, derivative, or mimetic inhibits histone acetylation by p300 thereby changing acetylation patterns. In some embodiments, the method further includes, activating autophagy pathways and promoting autophagosome formation.

In a further embodiment, the present disclosure is related to a composition for ameliorating aging or impacting the aging process to a patient subjected to environmental factors effecting the aging process. In some embodiments, the composition includes a synthetic lysine analog, derivative, or mimetic. In some embodiments, the synthetic lysine analog, derivative, or mimetic inhibits histone acetylation by p300 thereby changing acetylation patterns. In some embodiments, the synthetic lysine analog, derivative, or mimetic activates autophagy pathways to thereby promote autophagosome formation.

In another embodiment, the present disclosure relates to a method for a treatment of cancer, where the treatment includes inhibition of proliferation or survival of cancer cells. In some embodiments, the method includes administering a composition including a lysine analog, derivative, or mimetic. In some embodiments, the synthetic lysine analog, derivative, or mimetic interacts with the cancer cells to inhibit or prevent replication, survival, or formation of the cancer cells.

In an additional embodiment, the present disclosure relates to a method for prevention of cancer. In some embodiments, the method includes administering, prophylactically, a composition including a lysine analog, derivative, or mimetic. In some embodiments, the synthetic lysine analog, derivative, or mimetic interacts with potential cancer cells to inhibit or prevent formation of cancer cells.

In another aspect, the present disclosure relates to a composition for treatment or prevention of cancer. In some embodiments, the composition includes a synthetic lysine analog, derivative, or mimetic. In some embodiments, the synthetic lysine analog, derivative, or mimetic interacts with cancer cells to inhibit or prevent replication, survival, or formation of the cancer cells and inhibits proliferation or survival of the cancer cells.

### BRIEF DESCRIPTION OF THE DRAWINGS

A more complete understanding of the subject matter of the present disclosure may be obtained by reference to the following Detailed Description when taken in conjunction with the accompanying Drawings wherein:
FIG. 1 illustrates a 3-(4,5-dimethylthiazol-2-yl)-2,5-diphenyltetrazolium bromide (MTT) assay showing decreased viability of SH-SY5Y neuroblastoma and HCI-001 breast cancer cells in response to treatment with tranexamic acid.
FIG. 2 illustrates an MTT assay showing the reaction of multiple cancer cells (including SH-SY5Y and HCI-001) to tranexamic acid.
FIG. 3 illustrates an MTT assay showing that breast cancer cells (MCF10A) that had been altered to overexpress MYC were significantly more sensitive to tranexamic acid (growth was inhibited) than the same type of cell that had not been altered.
FIG. 4 illustrates the results on the effects of tranexamic acid (TA) on cancer cells and non-transformed epithelial cells.
FIG. 5 illustrates the results of MTT cell viability assays with increasing concentrations of tranexamic acid.
FIG. 6 illustrates MDA-MB-231 breast cancer cell extracts indicating that tranexamic acid blocks CDCP1 cleavage by preventing plasmin activation.
FIG. 7 illustrates data examining effects of tranexamic acid on histone lysine acetylation in a breast cancer cell line (BT474).
FIG. 8 illustrates that tranexamic acid decreases expression of the oncogenic protein epidermal growth factor receptor (EGFR) and reduce the levels of lysine acetylation of cellular proteins.
FIG. 9 illustrates that in MDA-MB-468 and BT474 breast cancer cells, 1% tranexamic acid elevates markers of the mitotic phase of the cell cycle.
FIG. 10 illustrates that tranexamic acid reduces protein lysine acetylation as early as 4 hours.
FIG. 11 illustrate results of enzyme assays examining the effects of tranexamic acid on the activity of the p300 acetyltransferase enzyme.
FIG. 12 illustrate results of enzyme assays examining the effects of tranexamic acid on the activity of the p300 acetyltransferase enzyme.
FIG. 13 illustrates that tranexamic acid suppresses tumor growth more effectively than vehicles when administered orally at a once daily dosage of 375 mg/kg.
FIG. 14 illustrates an analysis of tumor volumes in two treatment groups at treatment day 20 which demonstrates an eight-fold greater tumor volume in the vehicle group than the tranexamic acid treatment group.
FIG. 15 illustrates tranexamic acid suppresses AKT activity in both the MYC overexpressing MCF10A cells and the MDA-MB-468 human breast cancer cell line as indicated by reduced staining with the phospho-AKT substrate antibody
FIG. 16 illustrates tranexamic acid suppresses the activating tyrosine phosphorylation of the signal transducer and activator of transcription (STAT3) oncogene.
FIG. 17 illustrates MTT assays performed with tranexamic acid derivatives.
FIG. 18 illustrates high concentrations of a tranexamic acid derivative decreased programmed death-ligand 1 (PD-L1) expression

### DETAILED DESCRIPTION

It is to be understood that the following disclosure provides many different embodiments, or examples, for implementing different features of various embodiments. Specific examples of components and arrangements are described below to simplify the disclosure. These are, of course, merely examples and are not intended to be limiting. The section headings used herein are for organizational purposes and are not to be construed as limiting the subject matter described.

For proliferation and survival of diseases (*e.g.,* cancer), cells require protein-protein, protein-DNA, and protein-RNA interactions involving the three positively charged amino acids lysine, arginine, and histidine. These three positively charged amino acids (lysine, arginine, and histidine) provide the positive charge in the electrostatic bond between positively and negatively charged residues required in multiple protein-protein, protein-DNA, and protein-RNA connections involved in various biological activities such as, but not limited to, the proliferation and survival of pathogens and diseased cells (*e.g.,* cancer cells). These three amino acids have similar chemical structures. As such, adding supplemental amounts of one or more can block the activity of one or more of the amino acids that are not supplemented. Similarly, providing a sufficient amount of an appropriate synthetic analog, derivative, mimetic, analog prodrugs, derivative prodrugs, and/or mimetic prodrugs of lysine, arginine, or histidine can block the activity of one or more of the three amino acids. In brief, synthetic lysine analogs, derivatives, mimetics, lysine analog prodrugs, lysine derivative prodrugs, and/or lysine mimetic prodrugs (herein referred to as "lysine analogs") can disrupt the protein-protein, protein-DNA, and protein-RNA interactions by antagonizing lysine, arginine, and histidine residues on the proteins. For example, tranexamic acid, an example of a lysine analog, inhibits the growth of pathogens, such as, viruses, cancer cells, parasites, diseases, disease conditions, diseased cells, and the like, by antagonizing lysine to inhibit plasmin formation, similar to how it causes antifibrinolysis. As used herein, "lysine analogs" can refer to synthetic lysine analogs, derivatives, mimetics, lysine analog prodrugs, lysine derivative prodrugs, and/or lysine mimetic prodrugs, as mentioned above, and additionally, to a combination of the aforementioned and an additional positively charged amino acid, or refer to lysine (and forms of lysine) being substituted by an arginine or histidine analog or derivative, an amino acid derivative or analog with properties similar to, or comparable to, lysine.

With respect to specific diseases such as cancer, studies show that non-melanoma skin cancer induced by long-term ultraviolet (UV) irradiation in mice is reduced by the administration of a lysine analog, such as tranexamic acid. That is, cancer development is ameliorated by the administration of tranexamic acid. Tranexamic acid treatment suppresses increases in the plasma levels of matrix metalloproteinase-9 and interleukin (IL)-6, and skin expression of plasmin, CC chemokine 2, macrophages, signal transducer and activator of transcription (STAT3), cyclin D and vascular endothelial growth factor (VEGF)-A that occurred in mice subjected to long-term UV irradiation. This indicates that the non-melanoma skin cancer induced by long-term irradiation is ameliorated by tranexamic acid through regulation of the plasmin/macrophage/IL-6/STAT3/cyclin D signal transmission pathway. This ameliorative effect against skin cancer may be mediated via inhibition of the IL-6-induced expression of VEGF-A, and as such, the inhibition of plasmin formation reduces various biochemical effects that in turn reduce cancer formation. This illustrates the potential prophylactic use of lysine analogs in the prevention of cancer cell development.

Additionally, in diseased cells, such as cancer cells, plasmin cleaves enzymes that allow for cancer cell proliferation. Thus, by blocking the formation of plasmin by occupying the lysine binding spots on plasminogen and blocking plasminogen activators, lysine analogs inhibit the cleavage of CUB domain-containing protein 1 (CDCP1). CDCP1 is a transmembrane molecule that has been associated with cancer progression. Research identifying distinct molecules associated with a high risk of tumor metastasis has pointed to CDCP1 as a cancer-promoting molecule. CDCP1 is a transmembrane glycoprotein, high expression levels of which have been associated with colon cancer, breast cancer, prostate cancer, stomach cancer, lung cancer, kidney cancer, pancreas cancer, and skin cancer. Enhanced expression of CDCP1 in multiple types of cancer makes it an attractive therapeutic target.

*In vivo* findings have suggested that CDCP1 functions as an anti-apoptotic molecule facilitating survival of cancerous cells. Lysine analogs, such as tranexamic acid, inhibit the cleavage of CDCP1, which is required for cancer cell proliferation. This is of interest as various studies have indicated that blocking of CDCP1 cleavage prevents Akt-dependent survival and inhibits metastatic colonization through poly ADP-ribose polymerase 1 (PARP1) mediated apoptosis of cancer cells. Additionally, the transmembrane cell adhesion protein ADAM9 has been identified in cancer cell migration and lung cancer metastasis to the brain. Studies indicate that ADAM9 enhances the ability of tissue plasminogen activator (tPA) to cleave and stimulate the function of the promigratory protein CDCP1 to promote lung metastasis. Blocking this mechanism of cancer cell migration has been shown to prolong survival in tumor-bearing mice and has cooperated with other treatments to enhance cytotoxic treatment. Research shows ADAM9 regulates lung cancer metastasis to the brain by facilitating the tPA-mediated cleavage of CDCP1, this shows potential as a strategy to prevent or treat brain metastatic diseases such as cancer.

Moreover, it has been shown that specific cleavage of CDCP1, by plasmin-like serine proteases induces signal transduction that facilitates early stages of spontaneous metastasis leading to tumor cell intravasation, for example, cells escape from the primary tumor, stromal invasion occurs, or transendothelial migration occurs. Studies have identified that CDCP1 cleavage represents a therapeutic target for CDCP1-positive cancers. As such, the role of inhibiting the cleavage of CDCP1, required for cancer cell proliferation, represents a potential mechanism of action for treatment of cancer by inhibiting the ability of cancer cells to multiply. The contribution of lysine analogs in the ability to inhibit CDCP1 cleavage is of notable consideration as this plasmin-dependent pathway prevents cancer cell invasion and metastasis, thus providing for an anticancer effect.

Additionally, lysine analogs have the ability to act on specific proteins relevant to disease proliferation and survival (*e.g.,* cancer) by blocking the acetylation and/or methylation of lysine, arginine, or histidine residues. For example, lysine analogs, such as, for example, tranexamic acid, cause inhibition of lysine acetylation, which represents a mechanism of action where tranexamic acid inhibits acetylation by p300 histone acetyltransferase (p300). p300 is a large protein with multiple domains that bind to diverse proteins including many transcription factors. p300 is a lysine acetyltransferase that catalyzes the attachment of an acetyl group to lysine residues of histones and other cellular proteins. p300 catalyzed acetylation of histones and other proteins are utilized in gene activation with p300 being a coactivator of several transcription factors. Additionally, heightened p300 expression and related activities have been observed in various diseases, such as, but not limited to, cancer.

Histone acetylation is the process by which the lysine residues within the N-terminal tail protruding from the histone core of the nucleosome are acetylated as part of gene regulation. Histone acetylation plays a role in gene regulation, and the reactions are typically catalyzed by enzymes with histone acetyltransferase activity or histone deacetylase activity. Histone acetyltransferases are enzymes that acetylate conserved lysine amino acids on histone proteins by transferring an acetyl group from acetyl-CoA to form ε-N-acetyllysine. DNA is wrapped around histones, and by transferring an acetyl group to the histones, genes can be turned "on" or "off". In general, histone acetylation increases gene expression.

Acetylated histones represent a type of epigenetic marker within chromatin, a complex of DNA and protein found in eukaryotic cells. Acetylation removes the positive charge on the histones, thereby decreasing the interaction of the N termini of histones with the negatively charged phosphate groups of DNA. As a consequence, a condensed chromatin is transformed into a more relaxed structure that is associated with greater levels of gene transcription. The acetylation of lysine in the tails of histones in the nucleosome weakens the interaction of these histones with the DNA, enabling the activation of gene transcription. This relaxation can be reversed by histone deacetylase activity. Relaxed, transcriptionally active DNA is referred to as euchromatin, whereas more condensed DNA is referred to as heterochromatin. Condensation can be brought about by deacetylation and/or methylation processes.

Due to the regulatory role during transcription of epigenetic modifications in genes, changes in epigenetic markers, such as acetylation, can contribute to disease development (*e.g.,* cancer). Histone deacetylase expression and activity in diseased cells (*e.g.,* tumors) is different from normal cells. With respect to cancer, the overexpression and increased activity of histone deacetylase has been shown to be characteristic of tumorigenesis and metastasis, suggesting an important regulatory role of histone deacetylation on oncogene expression. Histone deacetylation alters the electrostatic properties of chromatin in a manner that favors the repression of gene transcription. Both histone acetylation and deacetylation are involved in many cellular signaling pathways and diseases, and given that lysine deacetylases regulates histone acetylation and gene expression, compounds that can inhibit the activity of these enzymes have many therapeutic benefits. Additionally, lysine acetylation regulates the activity of non-histone proteins in many cellular processes, including gene transcription, mRNA splicing, signal transduction, metabolism activity, and cell survival, making it an advantageous avenue for inhibiting proliferation and survival of diseased cells, that can cause, for example, neurological disorders, cardiovascular diseases, fatty liver diseases, metabolic diseases, non-alcoholic steatohepatitis, viral diseases, cancers, and the like.

With respect to cancer, one example is the regulation role of histone acetylation and deacetylation in p300, which contributes to oncogenesis. p300 is a coactivator of several oncogenic transcription factors, such as STAT3, NF-kB, and hypoxia-inducible factor-1a (HIF-1a). Genes regulated by these transcription factors are involved in cytokine-induced or hypoxia-induced cancer cell survival and sustained proliferation. Accordingly, inhibition of acetylation of p300 is an advantageous avenue for cancer treatment therapy. Furthermore, blood cancers, such as, but not limited to, lymphoma and leukemia, are sensitive to lysine acetyltrasferase activity and thus are likely targets. In some instances, brain cancer due to metastatic spread from the lungs in addition to lymphomas that originate in the primary central nervous system lymphoma (PCNSL) are also of particular interest.

Reversible lysine acetylation plays a regulatory role in various cellular signaling pathways and diseases. One example is the regulation role of histone acetylation and deacetylation by p300, which contributes to several diseases, including, but not limited to, hypertension, cardiovascular diseases, arrhythmia, heart failure, angiogenesis, liver diseases, cancers, and the like. p300 functions as histone acetyltransferase that regulates transcription of genes via chromatin remodeling by allowing histone proteins to wrap DNA less tightly. p300 also plays a role in regulating cell growth and division, which prompts cells to mature and assume specialized functions, and can also prevent the spread and growth of various cells. Accordingly, inhibition of acetylation by p300 is an advantageous avenue for disease treatment and prevention. Furthermore, indirect effects of p300 are readily envisioned.

Acetylation of proteins through interaction of enzymes, such as p300, with the lysine residues on those proteins has broad applicability to gene transcription and other activities that affect numerous diseases. As such, lysine analogs that cause inhibition of lysine acetylation, for example, lysine analogs that inhibit acetylation by p300, can prove beneficial in the treatment and prevention of numerous diseases that are effected by p300 activity. These diseases can include, without limitation, cardiovascular diseases, liver diseases, metabolic diseases, autoimmune diseases, neurological disorders, viral diseases, cancers, or combinations of the same and like. Both p300 and the CREB-binding protein (CBP) have been suggested to be good targets for disease ameliorating, and as such, therapeutics targeting these proteins via inhibition of lysine acetyltransferase provides for an advantageous mechanism of action in stopping disease proliferation and survival. p300 and CBP are very closely related lysine acetyltransferases, as such, principals and mechanisms of action that are applicable to lysine analogs and p300 generally apply to CBP as well.

Similarly, methylation on lysine, arginine, and histidine residues also relate to whether a particular histone and/or non-histone protein has stimulatory or inhibitory effects on gene expression. Histone methylation is a process by which methyl groups are transferred to amino acids of histone proteins that make up nucleosomes, which the DNA double helix wraps around to form chromatin. Methylation of histones can either increase or decrease transcription of genes, depending on which amino acids in the histones are methylated, and how many methyl groups are attached. Methylation events that weaken chemical attractions between histone tails and DNA increase transcription because they enable the DNA to uncoil from nucleosomes so that transcription factor proteins and RNA polymerase can access the DNA. This process is used in the regulation of gene expression that allows different cells to express different genes. As an example, protein lysine methylation is a post-translational modification that can regulate protein stability and function. This post-translational modification is regulated by lysine methyltransferases and lysine demethylases. In addition to histones, a great number of transcription factors are also methylated, often at multiple sites and to different degrees. Interfering with transcription factor lysine methylation can inhibit cell proliferation and survival, thereby reversing disease progression. As such, in addition to acetylation and deacetylation inhibitors, compositions that target lysine methyltransferases and lysine demethylases, for example, lysine analogs such as tranexamic acid, demonstrate an ability to ameliorate diseases and disease conditions.

As the acetylation and methylation of lysine residues regulates various activity of histones and non-histone proteins, such as inhibiting proliferation and survival of cells, it is further envisioned that the same effects can also be realized with the acetylation/deacetylation and methylation/demethylation of arginine and histidine residues of histones and non-histone proteins. Accordingly, an aspect of the present disclosure relates generally to ameliorating diseases and disease conditions utilizing lysine analogs, such as, but not limited to, tranexamic acid, via inhibiting at least one of lysine, arginine, and histidine acetylation and/or methylation. Additionally, an aspect of the present disclosure relates to the ability of lysine analogs to inhibit acetylation by the p300 enzyme to ameliorate various diseases and disease conditions.

A known function of p300 is its role in the nuclear localization of transcriptional regulatory proteins. In this role, p300 acetylates the N-terminal amino acid residues of certain transcriptional regulatory proteins and facilitates the localization and retention of the transcriptional regulatory proteins into and within the nucleus.

Additionally, non-enzymatic modifications of proteins can occur when a nucleophilic or redox-sensitive amino acid side chain encounters a reactive metabolite. The biological function of these modifications is typically limited by their irreversibility, and consequently these non-enzymatic modifications are often considered as indicators of diseases. Certain non-enzymatic modifications, however, can be reversed, which provides an additional layer of regulation and renders these modifications suitable for controlling a diverse set of cellular processes. For example, non-enzymatic modifications of lysine residues by acetyl-CoA or acetyl-phosphate can occur. This results in general blockage by a lysine analog, for example, tranexamic acid, of a lysine, arginine, or histidine residue on a protein involved in various biological processes, which can cause an ameliorating effect on various diseases and disease conditions.

Furthermore, similar to p300, MYC overexpression is found in various diseases (*e.g.,* cancer). The MYC transcription factor gene family includes MYC, MYCN, and MYCL1, and is involved in various cellular processes, such as, for example, cell proliferation, growth, apoptosis, and differentiation. A correlation between MYC gene family expression and progression of various diseases is found when the MYCN gene is amplified. For example, in diseases like cancer, similar to p300, there is an overexpression of MYC. In cancer, MYC is often constitutively and persistently expressed. This leads to the increased expression of many genes, some of which are involved in cell proliferation, contributing to the formation of diseased cells. The MYC oncogene plays a role in the development and progression of multiple cancers. Since MYC relies on p300 for its transcriptional activity and for its oncogenic functions within the nucleus, including the expression of telomerase that confers immortality upon cancer cells, MYC-positive tumors may be particularly sensitive to p300 inhibitors, such as lysine analogs. p300 can function as a shuttle to facilitate nuclear localization of other cargos using its lysine-rich nuclear localization sequence. Additionally, p300 can acetylate the lysine residues within specific proteins to regulate their translocation to the nucleus. Thus, lysine analogs can block the oncogenic functions of MYC by directly inhibiting p300 catalytic activity, preventing p300-dependent gene activation within the nucleus, blocking p300 and/or MYC translocation into the nucleus by antagonizing their nuclear localization sequences, or through more complicated mechanisms involving acetylation by p300 of MYC and its binding partner.

Recent studies indicate that similar interactions with MYC and/or p300 can be found in various diseases aside from cancer. For instance, p300 and MYC have been identified as being active in cardiovascular diseases as well as liver maladies, such as, but not limited to, nonalcoholic fatty liver disease. Due to the interactions of lysine analogs and MYC, lysine analogs exhibit characteristics for ameliorating diseases and disease conditions, thus indicating a broader reach than application to only cancerous cells. Furthermore, due to the effects of lysine analogs and its interactions as discussed herein, the method of action is further envisioned to effect protein synthesis in various manners due to the effects as outlined above.

Additionally, evidence suggests that many diseases (*e.g.,* cancer), produce immunosuppressive factors, and this has local as well as systemic effects on immune function. Plasmin, the effector protease of the fibrinolytic system, is recognized for its involvement in modulating immune function. Plasmin has been identified as a promoter of inflammation, though recent findings suggest a more complex role in modulation of immunity. These finding show that by the inhibition of plasmin, lysine analogs, such as tranexamic acid, enhance immune response.

Furthermore, in the absence of systemic hyperfibrinolysis, plasmin deficiency or blockades with lysine analogs, increase migration and proliferation of conventional dendritic cells and various antigen-presenting cells and T cells in the draining cervical lymph nodes after a traumatic brain injury. This suggests that lysine analogs, such as tranexamic acid, could also be clinically beneficial in modulating the inflammatory and immune response in patients suffering from various diseases and disease conditions (*e.g.,* cancer).

As lysine analogs have many advantageous properties in preventing formation and the proliferation and survival of diseases or disease conditions (*e.g.,* cancer), lysine analogs, such as tranexamic acid, can be used in combination with other therapeutic agents to combat various diseases and disease conditions. In some instances, it is advantageous to combine lysine analogs with various therapeutic agents with differing methods of action. In this manner, diseases, such as cancer, or disease conditions can be combatted via multiple modes and mechanisms. Additionally, other therapeutic agents can have varying activity in which their effects are complementary to lysine analogs. Furthermore, an additional therapeutic agent, when combined with lysine analogs, can provide for a lower dose of either the lysine analog or the additional therapeutic agent. In some instances, a combined effect can be greater than that predicted by individual potencies of each individual constituent, for example, either by requiring lower concentrations or by reacting more positively at similar concentrations. These interactions allow, for example, the use of lower concentrations of each constituent, a situation that can reduce adverse reactions of each individual constituent. As an example, lysine analogs could be combined with tannic acid or firsocostat in order to ameliorate diseases such as, but not limited to, fatty liver disease.

In addition, secondary effects of one of the constituents can enhance the primary effects of another one of the constituents, or provide other benefits, such as enhanced immune response and a larger decrease or cessation of disease proliferation. Additionally, co-therapies can be administered that can result in synergistic benefits, and can include, without limitation, immunotherapy or gene therapy, and chemotherapy. In various instances, these co-therapies can be performed separately. For example, chemotherapy infusions are commonly delivered in one day with a week or two delay before the next chemotherapy treatment. In this example, the lysine analogs could be administered during the days between the chemotherapy infusions. It is also envisioned that co-treatment could occur with immunotherapy, gene therapy, radiation therapy, surgical procedures, and combinations of the same and like. In embodiments where co-therapies and/or co-treatments are utilized, the lysine analogs can be delivered separately or as a single therapeutic dose. Furthermore, utilizing lysine analogs with various other constituents or co-therapies can have advantageous benefits. For example, utilizing lysine analogs with other constituents and co-therapies can result in the ability to avoid, or minimize, resistance being formed to each component of the drug by the disease. Furthermore, utilizing lysine analogs with other constituents and co-therapies can result in the ability to treat resistant strains of diseases, including those that are no longer effectively treated with one of the constituents or co-therapies.

Moreover, damage-associated molecular patterns, including mitochondrial DNA (mtDNA) are released during hemorrhage resulting in the development of endotheliopathy. Lysine analogs, such as tranexamic acid, are antifibrinolytic drugs and are typically used in hemorrhaging patients to enhance survival. Studies have shown that lysine analogs inhibit the release of endogenous mtDNA from granulocytes and endothelial cells. In addition, lysine analogs attenuate the loss of the endothelial monolayer integrity induced by exogenous mtDNA. Research demonstrate that lysine analogs strongly stimulate mitochondrial respiration and that lysine analogs can stimulate biogenesis of mitochondria and inhibit mitophagy.

These findings indicate additional secondary effects of lysine analogs as they have been shown to stimulate mitochondrial biogenesis, suppresses mitophagy, and strongly stimulate mitochondrial respiration, which could increase vascular cell and leukocyte survival and inhibit the release of mitochondrial damage-associated molecular patterns, providing an independent decrease in the development of inflammation. Research indicates that these effects of lysine analogs appear to be independent of its inhibition of plasmin formation.

Additionally, in some instances, more than one co-therapy can be administered with the lysine analogs presented herein. For example, lysine analogs, such as tranexamic acid, can be combined with an antimalarial therapeutic agent and/or an antibiotic agent. Moreover, lysine analogs, such as tranexamic acid, can be utilized with combinations of multiple antiviral therapies, for example, administration of two antiviral therapies and a lysine analog as disclosed herein. Additionally, lysine analogs, such as tranexamic acid, can be utilized in combination with anti-thrombotics medications and therapies, anti-seizure medications and therapies, blood thinners, such as apixaban, and combinations of the same and like. It is further envisioned that multiple lysine analogs can be administered concurrently. For example, a lysine analog such as tranexamic acid, can be administered in combination with another synthetic lysine analog, derivative, mimetic, and/or lysine analog prodrug, lysine derivative prodrug, and/or lysine mimetic prodrug. In various instances, multiple lysine analogs and multiple co-therapies can be administered. For example, two lysine analogs can be administered with two co-therapies, such as, for example, antimalarial therapies and antibiotic therapies.

Additionally, natural processes, such as aging, can contribute to disease development. Aging is characterized by the progressive loss of physiological function. The aging process can be modulated by environmental modifications, including diet, exercise, sun exposure, and stress management. However, recent observations indicate that inhibition of the histone acetyltransferase, p300, or the loss of p300 function, changes specific acetylation patterns and influences lifespan. These findings highlight the potential regulatory role of p300 on the aging process. Not only can this assist in regulating the aging process, but also this characteristic of p300 and aging can help prevent age-related diseases. Studies have indicated that compounds that act as an inhibitor of the epigenetic regulator p300 can play a role in longevity and lifespan. It has been demonstrated that compounds that inhibit p300 acetyltransferase and suppress acetylation of p300 targets in histones provide mechanistic insight into the role of p300 in longevity and increasing lifespan.

Aging is associated with an increase in age related diseases that include many cancers, neurodegenerative diseases, such as, for example, Parkinson's disease and Alzheimer's, atherosclerosis and associated cardiovascular disorders, such as heart attacks and stroke, macular degeneration, osteoarthritis, liver disease, osteoporosis, and sarcopenia. Aging, or its manifestations as chronic diseases of aging, can be slowed by a number of interventions that include diet, exercise, and even therapeutic agents.

Signs of aging include, without limitation, epigenetic alterations and deregulation of nutrient sensing. Levels of histone acetylation change during aging, and are typically related to an increase in acetyl-CoA levels. As acetyl-CoA is an acetyl group donor for protein acetylation reactions, its cellular level influences the activity of acetyltransferases. Particularly, histone and non-histone acetyltransferase p300 functions as a molecular sensor of changes in cellular acyl-CoA levels. Any decrease in cellular acetyl-CoA significantly limits the activity of p300 and decreases subsequent acetylation reactions involving certain cellular proteins. p300 blocks autophagy by acetylating autophagy related proteins. Autophagy is an evolutionary conserved recycling pathway responsible for the degradation, then turnover of cellular proteins and organelles, which is linked to the aging process. Without being bound by theory, it is believed that lysine analogs can inhibit p300 activity and suppress acetylation by p300 thereby exerting its effect on aging inhibition by activating autophagy pathways (which declines naturally during aging) and promoting autophagosome formation. In this manner, lysine analogs, and their role with p300, exhibit antiaging benefits in addition to ameliorating disease conditions. This can be manifested internally, on a cellular level (*e.g.* disease prevention) and on an external level (*e.g.* appearance).

As such, inhibition of p300 activity by lysine analogs, such as, tranexamic acid shows advantageous properties in prolonging lifespan, decreasing and/or slowing the aging process, decreasing and/or slowing manifestations of aging, reducing age-related diseases, or combinations of the same and like. Moreover, in view of the role p300 plays in the aging process, physical signs of aging, such as, but not limited to, wrinkles, skin lines, crow's feet, uneven and rough skin, tissue inflammation, tissue function loss, cellular degradation, and the like can be ameliorated via use of lysine analogs or therapeutic agents containing lysine analogs. For example, skin conditions caused by aging can utilize topical creams and moisturizing products having lysine analogs therein to slow, diminish, or reverse signs of aging. Additionally, lysine analogs or therapeutic agents including lysine analogs could be delivered to target sites on the skin to correct, for example, facial wrinkles and folds.

Reference will now be made to more specific embodiments of the present disclosure and data that provides support for such embodiments. However, it should be noted that the disclosure below is for illustrative purposes only and is not intended to limit the scope of the claimed subject matter in any way.

While various embodiments disclosed herein are directed to lysine analogs and their inhibition against cancer cells, it should be readily recognized that the general principals of the lysine analogs apply to a multitude of diseases. As such, one can readily envision ameliorating diseases and disease conditions that are effected by, for example, the ability of lysine analogs to inhibit p300, CBP, MYC, and other lysine acetyltransferases, the ability to inhibit lysine acetylation and/or deacetylation, including physical changes such as aging, methylation and/or demethylation on lysine and/or arginine residues, which also correlates to histones having stimulatory or inhibitory effects on gene expression, and non-enzymatic modification of lysine residues by acetyl-CoA or acetyl-phosphate, for example, general blockage by lysine analogs of the lysine, arginine, or histidine residues in proteins involved in biological processes that cause diseases, disease conditions, and the like.

These diseases can include, without limitation, liver diseases, non-alcoholic steatohepatitis, metabolic diseases, autoimmune diseases, cardiovascular diseases, hypertension, systemic hypertension, pulmonary arterial hypertension, vascular diseases, atherosclerosis, diabetic vascular diseases, vascular calcification, arrhythmias, heart failure, hypertrophy, fibrosis, cardiomyocyte proliferation and apoptosis, inflammation, diabetic cardiomyopathy, angiogenesis, neurological disorders, viral diseases, drug addiction, conditions caused by drug addiction, increased adipose tissue, insulin resistance, osteoporosis, or combinations of the same and like. Furthermore, given the properties of lysine analogs and p300, CBP, and/or MYC, one can readily envision that certain physiological properties, such as inhibition of p300 by tranexamic acid, extends to further roles relating to, for example, aging and disease conditions in which p300 plays a role in these biological process and/or pathways.

Accordingly, the following illustrates verification of tranexamic acid anticancer efficacy and the role of lysine acetyltransferase inhibition in its anticancer actions. Histone proteins reside in the cell nucleus and play roles in regulating gene expression. Whether histones have stimulatory or inhibitory effects on gene expression depends on their state of acetylation on lysine residues and methylation on lysine and/or arginine residues. Studies show that tranexamic acid decreases the acetylation of lysine residues in histone H3 in intact cells, and that tranexamic acid inhibits the lysine acetyltransferase, p300, in *in vitro* enzyme assays. Together, these results suggest that tranexamic acid blocks cancer cell viability by inhibiting lysine acetyltransferases in the nucleus, such as p300, by competing with the lysine binding site on acetyltransferases. This results in cancer cell death through altered gene expression. These properties are easily envisioned to apply to any disease or condition in which p300 acetyltransferase activity is inhibited, for example, cardiovascular diseases, liver diseases, aging, and the like as previously mentioned.

Studies have shown that tranexamic acid inhibits p300 acetyltransferase activity. Without being bound by theory, it is believed that tranexamic acid inhibits p300 by binding to the lysine binding pocket in its active site, and tranexamic acid may serve as a p300 substrate and become acetylated. Studies suggest that hematopoietic malignancies may be the most sensitive to p300 inhibition, so tranexamic acid may be particularly useful against these cancers. Tranexamic acid alters histone acetylation on lysine residues in cancer cells and this translates into changes in gene expression and the biological responses to tranexamic acid.

It should be noted that given the global nature of the control of biological processes by acetylation and its role in the histone code, the effect by tranexamic acid on protein acetylation has relevance to many diseases beyond cancer, for example, but not limited to, cardiovascular diseases, liver diseases, and the like as previously mentioned. Additionally, this role is extended to natural processes such as, for example, aging.

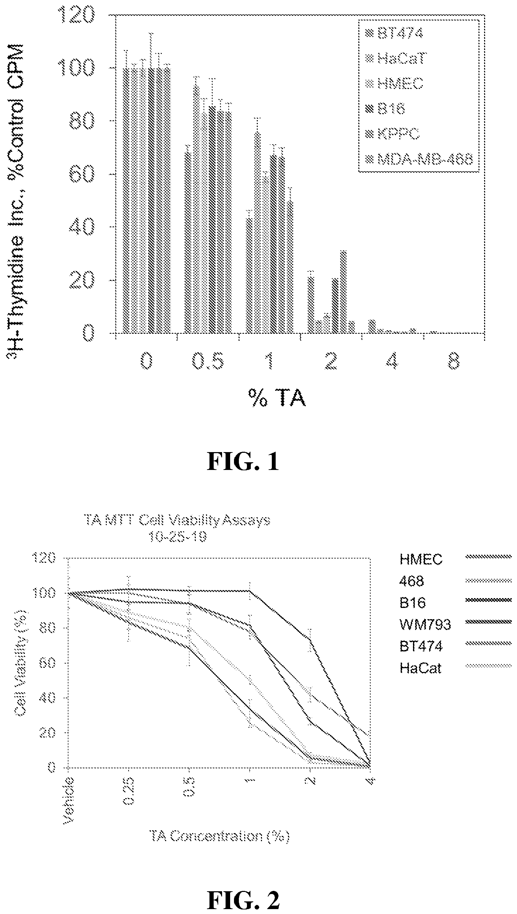

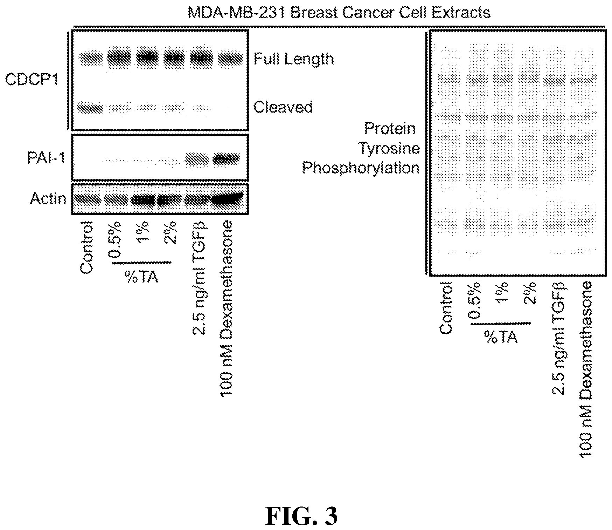

Research has found that one of the more sensitive cell lines to p300 inhibitors is SH-SY5Y. Tranexamic acid acts, in part, by inhibiting p300 and thus SH-SY5Y is sensitive to tranexamic acid. FIG. 1 illustrates a 3-(4,5-dimethylthiazol-2-yl)-2,5-diphenyltetrazolium bromide (MTT) assay showing decreased viability of SH-SY5Y neuroblastoma and HCI-001 breast cancer cells in response to treatment with tranexamic acid. FIG. 2 illustrates an MTT assay showing the reaction of multiple cancer cells (including SH-SY5Y and HCI-001) to tranexamic acid. The two cell lines that were most sensitive to tranexamic acid, MDA-MB-468, shown on the graph as 468, and SH-SY5Y, are also known to overexpress MYC. FIG. 3 illustrates an MTT assay showing that breast cancer cells (MCF10A) that had been altered to overexpress MYC were significantly more sensitive to tranexamic acid (growth was inhibited) than the same type of cell that had not been altered. Sensitivity of vector control MCF10A mammary epithelial cells or MCF10A cells overexpressing the MYC oncogene was examined, and interestingly, the MYC overexpressing cells were more sensitive than the control cells. As such, tumors that overexpress MYC are particularly sensitive to tranexamic acid.

The data shows that SH-SY5Y is the most sensitive to tranexamic acid when compared to other cell lines tested. As cancer cells that overexpress the MYC oncogene are more sensitive to tranexamic acid, MYC overexpression in SH-SY5Y was researched and it was identified that MYC is shown to be overexpressed in this cell line. This data confirms that cells overexpressing MYC are more sensitive to tranexamic acid. MYC is considered to be an un-druggable target, however in this case, data indicates that tranexamic acid acts in a "synthetic lethal" manner, in that it selectively kills cells with a tumor-specific genetic alteration, in this case MYC overexpression. This is of interest in that that tranexamic acid is more toxic to MYC overexpressing cells.

Together, these results provide a strong rationale for tranexamic acid, singularly or in use with additional therapeutic agents or co-therapies, to be utilized as an anticancer treatment. As discussed above, these results also support that disease conditions that share a common molecular driving force, for example, the driving force caused by p300 and MYC, or the over expression of p300 and MYC, can also benefit from tranexamic acid treatments. Data indicates that there is an interaction, physically and functionally, between p300 and MYC, which can include, without limitation, intracellular localization, as tranexamic acid works by antagonizing the nuclear localization signals of one or both proteins. Moreover, p300 and CBP are very closely related lysine acetyltransferases. As such, generally principles and mechanisms of actions that are applicable to tranexamic acid and p300 are envisioned to apply to CBP as well.

Based on the data presented herein, and in conjunction with further studies illustrated below, it can be concluded that if tranexamic acid inhibits p300 in tumors, that tranexamic acid additionally turns off regulatory T cells (Tregs), a subpopulation of T cells that modulate the immune system that are immunosuppressive. As such, inhibiting p300 can increase anticancer immunity. Furthermore, MYC suppresses the immune response as well. Thus, as tranexamic acid inhibits both p300 and MYC, tranexamic acid can activate the immune response resulting in additive, or synergistic, effects in combination with checkpoint inhibitors. Moreover, given the advantageous properties of tranexamic acid, it is further envisioned that the use and/or creation of an arginine analog, derivative, and/or mimetic that specifically targets the N terminal arginine residues on the histone, as tranexamic acid does on the lysine residues, would prove highly advantageous in ameliorating diseases and disease conditions along with slowing, or mitigating, aging and age-related diseases.

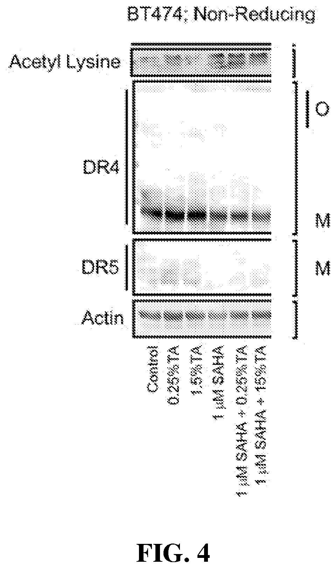

FIG. 4 illustrates the results on the effects of tranexamic acid (TA) on cancer cells and non-transformed epithelial cells. Tranexamic acid affected all the cell lines examined (breast cancer: BT474, MDA-MB-468; melanoma: B16; pancreatic cancer (mouse): KPPC; mammary epithelial: HMEC; and keratinocyte: HaCaT). Cells were treated for 24 h with tranexamic acid concentrations ranging from 0 to 8% and DNA synthesis during a 3 h window was measured. As illustrated in FIG. 4, the BT474 breast cancer cells were most sensitive to low concentrations of tranexamic acid, though tranexamic acid exhibited positive results across all cell lines. As indicated in FIG. 4, tranexamic acid also affects the proliferation of the non-transformed epithelial lines human mammary epithelia cells (HMEC) and human keratinocytes (HaCaT). The data shown in FIG. 4 represent the measurement of DNA synthesis which occurs during cell proliferation. Data indicated in FIG. 4 shows that tranexamic acid will likely exhibit stronger effects at lower concentrations when exposure time is increased.

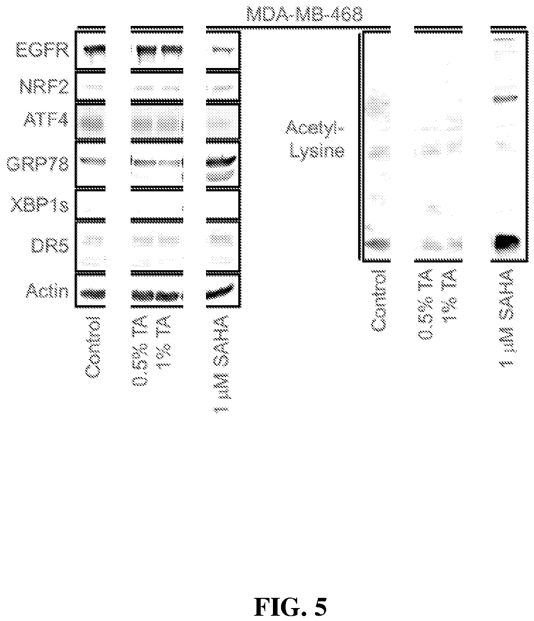

FIG. 5 illustrates an MTT cell viability assays with increasing concentrations of tranexamic acid. As shown in FIG. 5, the MTT results exhibit similar results as the effects of tranexamic acid on cancer cells and non-transformed epithelial cells, shown in FIG. 4, evaluating cell proliferation with respect to tranexamic acid concentrations and its effects on individual cell lines. FIG. 5 confirms that tranexamic acid dose dependently suppresses the viability of a broad array of cancer and epithelial cell lines.

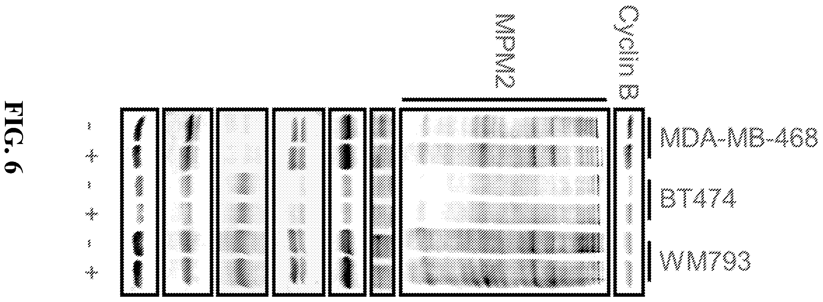

FIG. 6 illustrates MDA-MB-231 breast cancer cell extracts indicating that tranexamic acid blocks CDCP1 cleavage by preventing plasmin activation. The ability of tranexamic acid to inhibit CDCP1 cleavage is of value due to the abundance of evidence indicating the contribution of CDCP1 cleavage to cancer invasion and metastasis, as discussed above. Tranexamic acid acts in a manner distinct from, for example, TGF-β and glucocorticoids, which have previously been shown to block CDCP1 cleavage by upregulating PAI-1. Thus, while plasmin-dependent, these results indicate a novel anticancer effect exhibited by tranexamic acid, and FIG. 6 illustrates the effects of tranexamic acid on CDCP1 cleavage as a marker for plasmin activity.

As shown in FIG. 6, the lowest concentration of tranexamic acid (0.5%) produces good inhibition of CDCP1 cleavage. This, along with the MTT cell viability assays discussed above indicate that tranexamic acid blocks plasminogen activation at low concentrations, however acts on other cancer targets at higher concentrations.

Histone proteins bind DNA and control which genes are expressed. The ability of histones to control gene expression is understood in the histone code, in which modification of histone proteins by acetylation, methylation, and several other post-translational modifications control their activity. These modifications to histones and methylation of DNA itself are a part of epigenetic gene regulation, which refers to information that is not coded in the DNA itself, but is encoded by modifications to histones and DNA. A number of epigenetic drugs are either Food and Drug Administration (FDA)-approved for cancer or are under investigation. These include histone acetyltransferase inhibitors and histone deacetylases, both of which alter histone acetylation patterns.

Without being bound by theory, based on the data presented herein, tranexamic acid mimics lysine and arginine, and therefore competes for lysine and arginine residues in proteins that would normally be acetylated or methylated by acetyltransferases or methyltransferases. Stated in another way, tranexamic acid acts as a competitive inhibitor of lysine acetyltransferases, lysine methyltransferases, and arginine methyltransferases. Additionally, since acetylation and methylation of lysine and arginine residues in the histone proteins in the nucleus controls their interaction with DNA and alters gene expression, tranexamic acid has anticancer activity by acting through the histone code. Histone methylases and acetyltransferases are promising enzymes, and this is supported by the data presented herein showing that tranexamic acid reduces histone lysine acetylation.

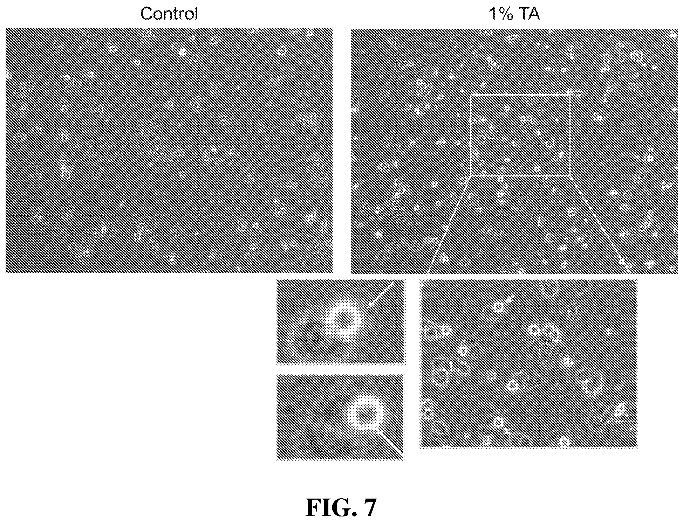

FIG. 7 illustrates experimental data examining effects of tranexamic acid on histone lysine acetylation in a second breast cancer cell line (BT474). The results are similar to previous data, in that tranexamic acid suppressed histone lysine acetylation, particularly at higher concentrations. As shown in FIG. 7, tranexamic acid did not overcome the effects of the histone deacetylase inhibitor SAHA. This data suggests that inhibition of lysine acetylation represents a new mechanism of action for tranexamic acid for preventing cancer cell proliferation.

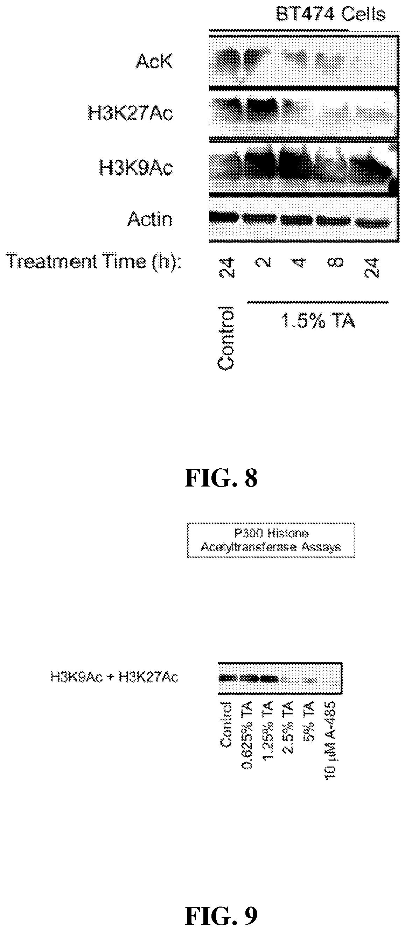

FIG. 8 illustrates that tranexamic acid decreases expression of the oncogenic protein epidermal growth factor receptor (EGFR), and tranexamic acid reduces the levels of lysine acetylation of cellular proteins. However, tranexamic acid does not increase markers of endoplasmic reticulum (ER) stress (ATF4, GRP78, and XBP1s), oxidative stress (NRF2), or death receptor 5 (DR5).

FIG. 9 illustrates that in MDA-MB-468 and BT474 breast cancer cells, 1% tranexamic acid elevates markers of the mitotic phase of the cell cycle, including cyclin B and MPM2. In addition, studies showed that tranexamic acid causes G2/M-phase cell cycle arrest. This study demonstrated that tranexamic acid causes accumulation of rounded cells that appear to be arrested in the mitotic phase of the cell cycle based on the presence of the "metaphase plate" of chromosomes.

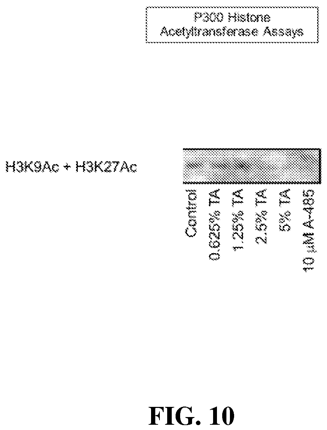

p300 and the CREB-binding protein (CBP) have been suggested to be good anticancer drug targets, and as such, anticancer therapeutics targeting these proteins via inhibition of lysine acetyltransferase provides for an advantageous mechanism of actions in stopping cancer cell proliferation. FIG. 10 illustrates that tranexamic acid reduces protein lysine acetylation as early as 4 h. Previous experiments on acetylation used MDA-MB-468 cell lines, and as such, FIG. 10 demonstrates inhibition of lysine acetylation by tranexamic acid in a second breast cancer cell line.

Furthermore, tranexamic acid blocks acetylation of the nuclear protein histone H3 on lysine 27 which is carried out by p300 and CBP. However, not on lysine 9, which is carried out by other enzymes not affected by tranexamic acid. Without being bound by theory, it is believed that tranexamic acid inhibits p300 acetyltransferase activity, confirming p300 as a direct target of tranexamic acid. This evidence suggests that tranexamic acid can provide for an enhanced inhibition of cancer cell proliferation.

FIG. 11 and FIG. 12 illustrate results of two sets of enzyme assays examining the effects of tranexamic acid on the activity of the p300 acetyltransferase enzyme. p300 transfers the acetyl group from acetyl CoA to the nitrogen of the lysine side chain in proteins. Both assays show that tranexamic acid inhibits p300 activity. This is measured by immunoblotting with antibodies that specifically recognize histone 3 (H3) acetylated (Ac) on lysine (K) number 9 or 27. A-485 is an established p300 inhibitor with anticancer activity in animal models, and was used in this assay as a positive control for acetyltransferase inhibition. In essence, the data presented herein illustrates that p300 is a direct tranexamic acid target and the inhibition of p300 enzyme activity by tranexamic acid explains many of the anticancer effects tranexamic acid exhibits.

The data in FIG. 11 and FIG. 12, in conjunction with the data presented herein shows that tranexamic acid inhibits protein lysine acetylation in cancer cells. This suggests that tranexamic acid inhibition of acetyltransferases is a previously unknown mechanism of tranexamic acid anticancer action. p300 is a global regulator of gene expression, and as such, this mechanism explains many of the non-plasmin effects of tranexamic acid.

In view of the aforementioned, synthetic lysine analogs, derivatives, mimetics, and/or lysine analog prodrugs ("lysine analogs") exhibit advantageous properties to inhibit the proliferation of cancer cells, and as such, can be utilized in the treatment and prevention of cancer singularly or in combination with other anticancer therapeutic agents and/or various co-therapies. As disclosed herein, a specific lysine analog, tranexamic acid, has been shown to disrupt protein-protein, protein-DNA, and protein-RNA interactions by antagonizing lysine, arginine, and histidine residues on the proteins. In addition, it has been demonstrated that tranexamic acid inhibits acetylation and methylation of lysine and arginine residues, and thus can stop cancer cell proliferation by inhibiting acetylation of the relevant histones. Furthermore, disclosed herein is data illustrating that tranexamic acid further inhibits the cleavage of CDCP1, which is needed for cancer cell proliferation, by occupying the lysine binding spots on plasminogen and blocking plasminogen activator.

Additionally, lysine analogs, such as, for example, tranexamic acid can be combined with other pharmaceutical agents to provide for a more effective cancer treatment regimen. This combination can prove to be especially effective when the immunosuppressive factors of lysine analogs are considered. Due to the inhibition of plasmin, lysine analogs, such as, for example, tranexamic acid enhances immune response. This can be especially beneficial in patients with compromised immune systems. As such, lysine analogs and other pharmaceutical agents, used in combination, can work in a synergistic combination as described herein.

As discussed herein, tranexamic acid is an anti-fibrinolytic drug that can be used to control excessive hemorrhaging. Tranexamic acid suppresses bleeding by preventing the conversion of plasminogen to plasmin, thus preventing fibrinolysis. Tranexamic acid exhibits anticancer activity *in vivo* and *in vitro.* Further, tranexamic acid anticancer actions involve novel mechanisms of action, as outlined in detail herein.

For example, tranexamic acid suppresses the growth of breast tumors in a patient-derived model of drug-resistant, HER2+, metastatic breast cancer (HCI-012/LVM2/LR10). As shown in FIG. 13, tranexamic acid suppresses tumor growth more effectively than a vehicle (water), when administered orally at a once daily dosage of 375 mg/kg. Analysis of tumor volumes in the two treatment groups at treatment day 20 demonstrates an eight-fold greater tumor volume in the vehicle group than the tranexamic acid treatment group (FIG. 14). The difference is statistically different with a P value of 0.035.

Furthermore, as discussed above, tranexamic acid suppresses the proliferation of multiple cancer and non-cancer cell lines, as measured by the incorporation of tritiated thymidine into DNA during cell division (FIG. 4). Analysis of the viability of a variety of cancer and non-cancer cell lines using the MTT assay shows that all lines are inhibited by tranexamic acid, although there is variable (FIG. 2). The most sensitive line to tranexamic acid is the SH-SY5Y neuroblastoma cell line that harbors amplification of the N-Myc oncogene.

The N-Myc-related gene c-Myc, or MYC, is frequently overexpressed in many human cancers, including breast cancer. Therefore, studies were conducted to identify if expressing the MYC oncogene in the MCF10A human mammary epithelial cell line alters the sensitivity of the cells to tranexamic acid. As shown in FIG. 3, MYC overexpression causes a significant increase in tranexamic acid sensitivity, suggesting that MYC overexpression may serve as a biomarker assay for the identification of tranexamic acid-responsive cancers. A more detailed mechanistic analysis was carried out to examine tranexamic acid effects on signaling pathways that contribute to cancer cell proliferation and survival. Tranexamic acid suppressed AKT activity in both the MYC overexpressing MCF10A cells and the MDA-MB-468 human breast cancer cell line as indicated by reduced staining with the phospho-AKT substrate antibody (arrows, FIG. 15). Tranexamic acid also reduced phosphorylation of the ribosomal S6 protein that is phosphorylated by the S6K1 enzyme. Further, tranexamic acid suppressed the activating tyrosine phosphorylation of the STAT3 oncogene (FIG. 16). Studies indicated that tranexamic acid: (1) reduces AKT-dependent phosphorylation of a subset of its substrates; (2) inhibits p70s6k, which phosphorylates the S6 protein-both AKT and p70s6k have basic residues in their consensus phosphorylation sites; and (3) decreases STAT3 phosphorylation on Tyrosine 705, but not Serine 727-Y705 phosphorylation of the STAT3 transcription factor is required for it to act as an oncogene. It was previously demonstrated that p300-dependent acetylation of STAT3 controls its tyrosine phosphorylation. This illustrates that tranexamic acid suppresses multiple oncogenic pathways that contribute to tumor growth and survival.

Additionally, tranexamic acid suppresses cleavage of the CDCP1 protein that is implicated in tumor invasion and metastasis. Studies showed that the plasmin enzyme cleaves CDCP1. The plasminogen activator inhibitor-1 (PAI-1) blocks CDCP1 cleavage. TGF-beta and the glucocorticoid dexamethasone suppress CDCP1 cleavage by increasing PAI-1 expression (FIG. 6). None of the treatments altered the overall pattern of protein tyrosine phosphorylation. Tranexamic acid treatment blocked CDCP1 cleavage without increasing PAI-1 levels. Tranexamic acid likely blocks CDCP1 cleavage by preventing plasminogen activation, although there is also evidence that tranexamic acid directly inhibits plasmin protease activity. Cleavage of CDCP1 may play a role in driving tumor invasion given the preferential binding of the cleaved form of CDCP1 with cell adhesion molecules such as beta1-integrin and E-cadherin. Consequently, tranexamic acid is predicted to block the pro-invasive functions of CDCP1 in cancer by preventing CDCP1 cleavage.

Furthermore, tranexamic acid suppresses histone acetylation (FIG. 8). Tranexamic acid prevents plasminogen activation by mimicking lysine and binding to lysine binding sites. Without being bound by theory, it is hypothesized that since lysine side chains are modified by acetylation by acetyltransferases that tranexamic acid may mimic lysine side chains and serve as an inhibitor of acetyltransferases. The p300 and CBP acetyltransferases in particular play roles in the control of global gene expression and have been identified as therapeutic targets for anticancer drugs. Tranexamic acid suppressed histone acetylation compared with the vehicle treatment (control). Tranexamic acid did not increase markers of endoplasmic reticulum stress markers (ATF4, GRP78, and XBP1s), the oxidative stress marker NRF2, death receptor 5 (DR5) upregulation, or EGFR downregulation.

Studies showed that topical administration of tranexamic acid induces death of melanoma cells adjacent to the site of administration. Mice bearing B 16 murine melanoma tumors were treated topically once daily for five consecutive days with the tranexamic acid vehicle and 20% tranexamic acid. Tumors were collected and tumor sections were stained with hematoxylin and eosin. Tranexamic acid killed melanoma cells adjacent to the skin, with less evidence of melanoma death further from the skin. In contrast, vehicle-treated tumors did not show evidence of necrosis adjacent to the skin. These results provide evidence that tranexamic acid may be useful for the topical treatment of small melanomas or pre-melanocytic nevi.

With respect to tranexamic acid derivatives, MTT assays were performed with tranexamic acid derivatives (FIG. 17). MDA-MB 468 cells were plated at 6,250 cells per well in a 96-well plate and incubated overnight at 37 °C. Cells were subsequently incubated for 72 h with increasing concentrations of tranexamic acid (TA), TA-NAc, or TA-R2 at 37°C. Cell viability was evaluated using an MTT assay. The results are represent the mean ±SD (standard deviation) of ten replicate determinations for each concentration tested. The data represents an arginine analog (based on tranexamic acid for efficiency) to see if greater activity than traditional tranexamic acid is observed. Both tranexamic acid and tranexamic acid with derivatives reduced MDA-MB 468 cell viability. FIG. 18 illustrates that at high concentration (2%) TA-R2 decreased programmed death-ligand 1 (PD-L1) expression, while tranexamic acid at the same concentration did not. This data provides justification for using a synthetic arginine analog against certain cancers or other diseases where it may be more effective than a lysine analog (because the residues being antagonized are arginine or are otherwise more effectively blocked by an arginine analog than a lysine or histidine analog), and for combining a synthetic arginine analog with a synthetic lysine analog for synergistic effect.

In view of the aforementioned, in an embodiment, the present disclosure relates to a method of ameliorating or preventing a disease or disease condition. In some embodiments, the method includes administering a composition having a synthetic lysine analog, derivative, or mimetic. In some embodiments, the synthetic lysine analog, derivative, or mimetic interacts with cells associated with the disease or disease condition to inhibit or prevent replication, survival, or formation of the cells.

In some embodiments, the method includes disrupting, by the composition, at least one of protein-protein, protein-DNA, and protein-RNA interaction by antagonizing at least one of lysine, arginine, and histidine residues on a protein. In some embodiments, the method includes blocking, by the composition, directly or indirectly at least one of acetylation, deacetylation, methylation, and demethylation processes of at least one of a lysine, arginine, or histidine residue of at least one of a histone or non-histone protein. In some embodiments, the blocking results in acetylation inhibition of at least one of p300 histone acetyltransferase, a CREB-binding protein (CBP), and MYC. In some embodiments, the disease or disease condition is cause by an overexpression of at least one of p300 or MYC. In some embodiments, the method includes modifying, by the composition, at least one of a lysine, arginine, and histidine residue of a protein by at least one of acetyl-CoA and acetyl-phosphate to thereby block a binding site on the protein. In some embodiments, the method includes occupying, by the composition, a lysine binding spot on plasminogen and blocking formation of plasmin. In some embodiments, the method includes modulating, by the composition, inflammatory and immune response in patients to thereby ameliorate the disease or disease condition. In some embodiments, the compositions provides improvements in delivery, stimulates mitochondrial biogenesis, suppresses mitophagy, or stimulates mitochondrial respiration. In some embodiments, the improvement in delivery is improved cellular penetration.

In some embodiments, the synthetic lysine analog, derivative, or mimetic can include, without limitation, of tranexamic acid, a combination of tranexamic acid and an additional positively charged amino acid, epsilon-aminocaproic acid (EACA), and AZD 6564, or substituted by a natural or synthetic arginine or histidine analog or derivative, or another amino acid derivative or analog. In some embodiments, the disease can include, without limitation, of liver diseases, non-alcoholic steatohepatitis, metabolic diseases, autoimmune diseases, cardiovascular diseases, hypertension, systemic hypertension, pulmonary arterial hypertension, vascular diseases, atherosclerosis, diabetic vascular diseases, vascular calcification, arrhythmias, heart failure, hypertrophy, fibrosis, cardiomyocyte proliferation and apoptosis, inflammation, diabetic cardiomyopathy, angiogenesis, neurological disorders, viral diseases, drug addiction, conditions caused by drug addiction, increased adipose tissue, insulin resistance, osteoporosis, and combinations thereof.

In some embodiments, the composition is in a solution. In some embodiments, the solution is adapted to be delivered orally to accommodate greater concentrations of the synthetic lysine analog, derivative, or mimetic. In some embodiments, the solution has a concentration of about 1 to about 60% by weight of the synthetic lysine analog, derivative, or mimetic. In some embodiments, the solution has a concentration of about 1 to about 30% by weight of the synthetic lysine analog, derivative, or mimetic. In some embodiments, the synthetic lysine analog, derivative, or mimetic is applied as part of a vehicle which adapts to human skin, is applied via inhalation, is applied via nebulization methods, is applied via a spray, is applied via a nasal spray, and combinations thereof. In some embodiments, the solution containing the synthetic lysine analog, derivative, or mimetic is administered intravenously. In some embodiments, the solution is administered directly to an area of interest or administered to an area and transported to the area of interest. In some embodiments, the area of interest can include, without limitation, of an organ, a tumor, a diseased location, a malady location, the olfactory nerve and subsequently the brain, and combinations thereof. In some embodiments, the solution is delivered to an area via a percutaneous needle injection into an area of interest. In some embodiments, the area of interest can include, without limitation, of an organ, a tumor, a diseased location, a malady location, and combinations thereof. In some embodiments, the method includes measuring, in real-time, a target location of the area of interest to deliver the percutaneous needle injection.

In some embodiments, the solution is applied via a vehicle that allows the synthetic lysine analog, derivative, or mimetic to be delivered in a time-released fashion. In some embodiments, the composition is delivered orally, delivered via tablet or capsule, sublingually, via buccal delivery, or via mucosal delivery. In some embodiments, the composition is administered from about 1 mg/kg to about 1000 mg/kg. In some embodiments, the composition is administered from about 20 mg/kg to about 200 mg/kg. In some embodiments, the composition is administered from about 100 mg/kg to about 1000 mg/kg. In some embodiments, doses can range up to about 10000 mg/kg. In some embodiments, the composition is administered one time daily, two times daily, three times daily, four times daily, five times daily, or more. In some embodiments, oral administration includes administration of a first tablet followed by a second tablet. In some embodiments, the second tablet is breakable in at least one location for continued administration. In some embodiments the composition is administered for a prescribed period of time, until the disease or disease condition is cured, continuously until end of life to treat, avoid or inhibit the disease or disease condition.

In some embodiments, the composition is delivered in a time-released fashion. In some embodiments, the administering of the composition occurs at least once a day, at least once every two days, at least once every three days, at least once every four days, at least once every five days, at least once every six days, at least once a week, at least once two weeks, at least once every three weeks, at least once every few weeks. In some embodiments, the composition is administered by at least one of a mechanical device, a permanent or resorbable material, and a vehicle whereby the synthetic lysine analog, derivative, or mimetic is delivered in a time-released fashion. In some embodiments, the composition is administered via a transdermal patch. In some embodiments, the composition is administered via an injected or implanted liposomal delivery depot. In some embodiments, the composition includes ingredients that provide for rapid systemic penetration or extended release.

In some embodiments, the administering is performed systemically. In some embodiments, the composition is administered from about 1 mg/kg to about 100 mg/kg every 6-8 hours. In some embodiments, the composition includes an additional therapeutic agent. In some embodiments, the additional therapeutic agent has a mechanism of action complementary to the composition having the synthetic lysine analog, derivative, or mimetic. In some embodiments, the synthetic lysine analog, derivative, or mimetic is at least one of a lysine prodrug, a lysine analog prodrug, a synthetic lysine analog prodrug, a lysine derivative prodrug, and a lysine mimetic prodrug. In some embodiments, the method includes administering a co-therapy in conjunction with the composition. In some embodiments, the co-therapy results in synergistic benefits. In some embodiments, the co-therapy can include, without limitation, of immunotherapy, gene therapy, chloroquine, chemotherapy, monoclonal antibodies or antibody therapies, radiation, radiation therapy, electric fields, temperature therapies, surgical intervention, therapeutic treatments, ultrasonic therapy, ultrasound therapy, and combinations thereof. In some embodiments, the administering of the composition is conducted between multiple co-therapy treatments. In some embodiments, the administering of the composition is delivered separately or as a single therapy dose.

In another embodiment, the present disclosure relates to a composition for ameliorating or preventing a disease or disease condition. In some embodiments, the composition includes a synthetic lysine analog, derivative, or mimetic. In some embodiments, the synthetic lysine analog, derivative, or mimetic interacts with cells associated with the disease or disease condition to inhibit or prevent replication, survival, or formation of the cells.

In some embodiments, the synthetic lysine analog, derivative, or mimetic disrupts at least one of protein-protein, protein-DNA, and protein-RNA interaction by antagonizing at least one of lysine, arginine, and histidine residues on a protein. In some embodiments, the synthetic lysine analog, derivative, or mimetic blocks directly or indirectly at least one of acetylation, deacetylation, methylation, and demethylation processes of at least one of a lysine, arginine, or histidine residue of at least one of a histone or non-histone protein. In some embodiments, the blocking results in acetylation inhibition of at least one of p300 histone acetyltransferase, a CREB-binding protein (CBP), and MYC. In some embodiments, the disease or disease condition is caused by an overexpression of at least one of p300 or MYC. In some embodiments, the synthetic lysine analog, derivative, or mimetic modifies at least one of a lysine, arginine, and histidine residue of a protein by at least one of acetyl-CoA and acetyl-phosphate to thereby block a binding site on the protein. In some embodiments, the synthetic lysine analog, derivative, or mimetic occupies a lysine binding spot on plasminogen and blocking formation of plasmin. In some embodiments, the synthetic lysine analog, derivative, or mimetic modulates inflammatory and immune response in patients to thereby ameliorate the disease or disease condition. In some embodiments, the compositions provides improvements in delivery, stimulates mitochondrial biogenesis, suppresses mitophagy, or stimulates mitochondrial respiration. In some embodiments, the improvement in delivery is improved cellular penetration.

In some embodiments, the synthetic lysine analog, derivative, or mimetic can include, without limitation, of tranexamic acid, a combination of tranexamic acid and an additional positively charged amino acid, epsilon-aminocaproic acid (EACA), and AZD 6564, or substituted by a natural or synthetic arginine or histidine analog or derivative, or another amino acid derivative or analog. In some embodiments, the disease can include, without limitation, of liver diseases, non-alcoholic steatohepatitis, metabolic diseases, autoimmune diseases, cardiovascular diseases, hypertension, systemic hypertension, pulmonary arterial hypertension, vascular diseases, atherosclerosis, diabetic vascular diseases, vascular calcification, arrhythmias, heart failure, hypertrophy, fibrosis, cardiomyocyte proliferation and apoptosis, inflammation, diabetic cardiomyopathy, angiogenesis, neurological disorders, viral diseases, drug addiction, conditions caused by drug addiction, increased adipose tissue, insulin resistance, osteoporosis, and combinations thereof. In some embodiments, the synthetic lysine analog, derivative, or mimetic is in a solution. In some embodiments, the solution is adapted to be delivered orally to accommodate greater concentrations of the synthetic lysine analog, derivative, or mimetic. In some embodiments, the solution has a concentration of about 1 to about 60% by weight of the synthetic lysine analog, derivative, or mimetic. In some embodiments, the solution has a concentration of about 1 to about 30% by weight of the synthetic lysine analog, derivative, or mimetic. In some embodiments, the solution is applied as part of a vehicle which adapts to human skin, is applied via inhalation, is applied via nebulization methods, is applied via a spray, is applied via a nasal spray, and combinations thereof. In some embodiments, the solution containing the synthetic lysine analog, derivative, or mimetic is administered intravenously. In some embodiments, the solution is administered directly to an area of interest or administered to an area and transported to the area of interest. In some embodiments, the area of interest can include, without limitation, of an organ, a tumor, a diseased location, a malady location, the olfactory nerve and subsequently the brain, and combinations thereof. In some embodiments, the solution is delivered to an area via a percutaneous needle injection into an area of interest. In some embodiments, the area of interest can include, without limitation, of an organ, a tumor, a diseased location, a malady location, and combinations thereof. In some embodiments, a target location of the area of interest is monitored in real-time to deliver the percutaneous needle injection.

In some embodiments, the solution is applied via a vehicle that allows the synthetic lysine analog, derivative, or mimetic to be delivered in a time-released fashion. In some embodiments, the synthetic lysine analog, derivative, or mimetic is delivered orally, delivered via tablet or capsule, sublingually, via buccal delivery, or via mucosal delivery. In some embodiments, the synthetic lysine analog, derivative, or mimetic is administered from about 1 mg/kg to about 1000 mg/kg. In some embodiments, the synthetic lysine analog, derivative, or mimetic is administered from about 20 mg/kg to about 200 mg/kg. In some embodiments, the synthetic lysine analog, derivative, or mimetic is administered from about 100 mg/kg to about 1000 mg/kg. In some embodiments, doses can range up to about 10000 mg/kg. In some embodiments, the synthetic lysine analog, derivative, or mimetic is administered one time daily, two times daily, three times daily, four times daily, five times daily, or more. In some embodiments, oral administration of the synthetic lysine analog, derivative, or mimetic comprises administration of a first tablet followed by a second tablet. In some embodiments, the second tablet is breakable in at least one location for continued administration. In some embodiments, the synthetic lysine analog, derivative, or mimetic is administered for a prescribed period of time, until the disease or disease condition is cured, continuously until end of life to treat, avoid or inhibit the disease or disease condition.

In some embodiments, the synthetic lysine analog, derivative, or mimetic is delivered in a time-released fashion. In some embodiments, the synthetic lysine analog, derivative, or mimetic is administered at least once a day, at least once every two days, at least once every three days, at least once every four days, at least once every five days, at least once every six days, at least once a week, at least once two weeks, at least once every three weeks, at least once every few weeks. In some embodiments, the synthetic lysine analog, derivative, or mimetic is administered by at least one of a mechanical device, a permanent or resorbable material, and a vehicle whereby the synthetic lysine analog, derivative, or mimetic is delivered in a time-released fashion. In some embodiments, the synthetic lysine analog, derivative, or mimetic is administered via a transdermal patch. In some embodiments, the synthetic lysine analog, derivative, or mimetic is administered via an injected or implanted liposomal delivery depot. In some embodiments, the composition further includes ingredients that provide for rapid systemic penetration or extended release.

In some embodiments, the synthetic lysine analog, derivative, or mimetic is administered systemically. In some embodiments, the synthetic lysine analog, derivative, or mimetic is administered from about 1 mg/kg to about 100 mg/kg every 6-8 hours. In some embodiments, the composition further includes an additional therapeutic agent. In some embodiments, the additional therapeutic agent has a mechanism of action complementary to the synthetic lysine analog, derivative, or mimetic. In some embodiments, the synthetic lysine analog, derivative, or mimetic is at least one of a lysine prodrug, a lysine analog prodrug, a synthetic lysine analog prodrug, a lysine derivative prodrug, and a lysine mimetic prodrug. In some embodiments, the synthetic lysine analog, derivative, or mimetic is administered in conjunction with a co-therapy. In some embodiments, the co-therapy results in synergistic benefits. In some embodiments, the co-therapy can include, without limitation, of immunotherapy, gene therapy, chloroquine, chemotherapy, monoclonal antibodies or antibody therapies, radiation, radiation therapy, electric fields, temperature therapies, surgical intervention, therapeutic treatments, ultrasonic therapy, ultrasound therapy, and combinations thereof. In some embodiments, the administration of the synthetic lysine analog, derivative, or mimetic is conducted between multiple co-therapy treatments. In some embodiments, the administration of the synthetic lysine analog, derivative, or mimetic is delivered separately or as a single therapy dose.

In an additional embodiment, the present disclosure relates to a method of ameliorating aging or impacting the aging process to a patient subjected to environmental factors effecting the aging process. In some embodiments, the method includes administering a composition having a synthetic lysine analog, derivative, or mimetic. In some embodiments, the synthetic lysine analog, derivative, or mimetic inhibits histone acetylation by p300 thereby changing acetylation patterns. In some embodiments, the method further includes, activating autophagy pathways and promoting autophagosome formation.

In some embodiments, antiaging benefits are manifested internally on a cellular level. In some embodiments, antiaging benefits are manifested externally via appearance of skin. In some embodiments, the synthetic lysine analog, derivative, or mimetic can include, without limitation, of tranexamic acid, a combination of tranexamic acid and an additional positively charged amino acid, epsilon-aminocaproic acid (EACA), and AZD 6564, or substituted by a natural or synthetic arginine or histidine analog or derivative, or another amino acid derivative or analog. In some embodiments, the composition is in a solution. In some embodiments, the solution is adapted to be delivered orally to accommodate greater concentrations of the synthetic lysine analog, derivative, or mimetic. In some embodiments, the solution has a concentration of about 1 to about 60% by weight of the synthetic lysine analog, derivative, or mimetic. In some embodiments, the solution has a concentration of about 1 to about 30% by weight of the synthetic lysine analog, derivative, or mimetic. In some embodiments, the synthetic lysine analog, derivative, or mimetic is applied as part of a vehicle which adapts to human skin, is applied via inhalation, is applied via nebulization methods, is applied via a spray, is applied via a nasal spray, and combinations thereof.

In some embodiments, the solution containing the synthetic lysine analog, derivative, or mimetic is administered intravenously. In some embodiments, the solution is administered directly to an area of interest or administered to an area and transported to the area of interest. In some embodiments, the area of interest can include, without limitation, of an organ, a tumor, a diseased location, a malady location, the olfactory nerve and subsequently the brain, and combinations thereof. In some embodiments, the solution is delivered to an area via a percutaneous needle injection into an area of interest. In some embodiments, the area of interest can include, without limitation, of an organ, a tumor, a diseased location, a malady location, and combinations thereof. In some embodiments, the method includes measuring, in real-time, a target location of the area of interest to deliver the percutaneous needle injection. In some embodiments, the solution is applied via a vehicle that allows the synthetic lysine analog, derivative, or mimetic to be delivered in a time-released fashion. In some embodiments, the composition is delivered orally, delivered via tablet or capsule, sublingually, via buccal delivery, or via mucosal delivery. In some embodiments, the composition is administered from about 1 mg/kg to about 1000 mg/kg. In some embodiments, the composition is administered from about 20 mg/kg to about 200 mg/kg. In some embodiments, the composition is administered from about 100 mg/kg to about 1000 mg/kg. In some embodiments, doses can range up to about 10000 mg/kg. In some embodiments, the composition is administered one time daily, two times daily, three times daily, four times daily, five times daily, or more. In some embodiments, oral administration includes administration of a first tablet followed by a second tablet. In some embodiments, the second tablet is breakable in at least one location for continued administration. In some embodiments the composition is administered for a prescribed period of time to provide antiaging benefits or continuously until end of life to provide antiaging benefits.

In some embodiments, the composition is delivered in a time-released fashion. In some embodiments, the administering of the composition occurs at least once a day, at least once every two days, at least once every three days, at least once every four days, at least once every five days, at least once every six days, at least once a week, at least once two weeks, at least once every three weeks, at least once every few weeks. In some embodiments, the composition is administered by at least one of a mechanical device, a permanent or resorbable material, and a vehicle whereby the synthetic lysine analog, derivative, or mimetic is delivered in a time-released fashion. In some embodiments, the composition is administered via a transdermal patch. In some embodiments, the composition is administered via an injected or implanted liposomal delivery depot. In some embodiments, the composition includes ingredients that provide for rapid systemic penetration or extended release. In some embodiments, the administering is performed systemically. In some embodiments, the composition is administered from about 1 mg/kg to about 100 mg/kg every 6-8 hours.

In a further embodiment, the present disclosure is related to a composition for ameliorating aging or impacting the aging process to a patient subjected to environmental factors effecting the aging process. In some embodiments, the composition includes a synthetic lysine analog, derivative, or mimetic. In some embodiments, the synthetic lysine analog, derivative, or mimetic inhibits activity of histone acetyltransferase, p300, thereby changing acetylation patterns. In some embodiments, the synthetic lysine analog, derivative, or mimetic activates autophagy pathways to thereby promote autophagosome formation.

In some embodiments, antiaging benefits are manifested internally on a cellular level. In some embodiments, antiaging benefits are manifested externally via appearance of skin. In some embodiments, the synthetic lysine analog, derivative, or mimetic can include, without limitation, of tranexamic acid, a combination of tranexamic acid and an additional positively charged amino acid, epsilon-aminocaproic acid (EACA), and AZD 6564, or substituted by a natural or synthetic arginine or histidine analog or derivative, or another amino acid derivative or analog. In some embodiments, the synthetic lysine analog, derivative, or mimetic is in a solution. In some embodiments, the solution is adapted to be delivered orally to accommodate greater concentrations of the synthetic lysine analog, derivative, or mimetic. In some embodiments, the solution has a concentration of about 1 to about 60% by weight of the synthetic lysine analog, derivative, or mimetic. In some embodiments, the solution has a concentration of about 1 to about 30% by weight of the synthetic lysine analog, derivative, or mimetic. In some embodiments, the solution is applied as part of a vehicle which adapts to human skin, is applied via inhalation, is applied via nebulization methods, is applied via a spray, is applied via a nasal spray, and combinations thereof. In some embodiments, the solution is administered intravenously. In some embodiments, the solution is administered directly to an area of interest or administered to an area and transported to the area of interest. In some embodiments, the area of interest can include, without limitation, of an organ, a tumor, a diseased location, a malady location, the olfactory nerve and subsequently the brain, and combinations thereof. In some embodiments, the solution is delivered to an area via a percutaneous needle injection into an area of interest. In some embodiments, the area of interest can include, without limitation, of an organ, a tumor, a diseased location, a malady location, and combinations thereof. In some embodiments, a target location of the area of interest is monitored in real-time to deliver the percutaneous needle injection. In some embodiments, the solution is applied via a vehicle that allows the synthetic lysine analog, derivative, or mimetic to be delivered in a time-released fashion.

In some embodiments, the synthetic lysine analog, derivative, or mimetic is delivered orally, delivered via tablet or capsule, sublingually, via buccal delivery, or via mucosal delivery. In some embodiments, the synthetic lysine analog, derivative, or mimetic is administered from about 1 mg/kg to about 1000 mg/kg. In some embodiments, the synthetic lysine analog, derivative, or mimetic is administered from about 20 mg/kg to about 200 mg/kg. In some embodiments, the synthetic lysine analog, derivative, or mimetic is administered from about 100 mg/kg to about 1000 mg/kg. In some embodiments, doses can range up to about 10000 mg/kg. In some embodiments, the synthetic lysine analog, derivative, or mimetic is administered one time daily, two times daily, three times daily, four times daily, five times daily, or more. In some embodiments, oral administration of the synthetic lysine analog, derivative, or mimetic comprises administration of a first tablet followed by a second tablet. In some embodiments, the second tablet is breakable in at least one location for continued administration. In some embodiments, the synthetic lysine analog, derivative, or mimetic is administered for a prescribed period of time to provide antiaging benefits or continuously until end of life to provide antiaging benefits.

In some embodiments, the synthetic lysine analog, derivative, or mimetic is delivered in a time-released fashion. In some embodiments, the synthetic lysine analog, derivative, or mimetic is administered at least once a day, at least once every two days, at least once every three days, at least once every four days, at least once every five days, at least once every six days, at least once a week, at least once two weeks, at least once every three weeks, at least once every few weeks.

In some embodiments, the synthetic lysine analog, derivative, or mimetic is administered by at least one of a mechanical device, a permanent or resorbable material, and a vehicle whereby the synthetic lysine analog, derivative, or mimetic is delivered in a time-released fashion. In some embodiments, the synthetic lysine analog, derivative, or mimetic is administered via a transdermal patch. In some embodiments, the synthetic lysine analog, derivative, or mimetic is administered via an injected or implanted liposomal delivery depot. In some embodiments, the composition further includes ingredients that provide for rapid systemic penetration or extended release. In some embodiments, the synthetic lysine analog, derivative, or mimetic is administered systemically. In some embodiments, the synthetic lysine analog, derivative, or mimetic is administered from about 1 mg/kg to about 100 mg/kg every 6-8 hours.

In some embodiments, the present disclosure pertains to a method for a treatment of cancer, where the treatment includes inhibition of proliferation or survival of cancer cells. In some embodiments, the method includes administering a composition including a synthetic lysine analog, derivative, or mimetic. In some embodiments, the synthetic lysine analog, derivative, or mimetic interacts with the cancer cells to inhibit or prevent replication, survival, or formation of the cancer cells.

In some embodiments, the method includes disrupting, by the composition, at least one of protein-protein, protein-DNA, and protein-RNA interaction by antagonizing at least one of lysine, arginine, and histidine residues on a protein. In some embodiments, the method includes blocking, by the composition, directly or indirectly acetylation or methylation of lysine or arginine residues. In some embodiments, the blocking results in acetylation inhibition of at least one of a p300 histone and a CREB-binding protein (CBP). In some embodiments, the method includes blocking formation of plasmin to thereby inhibit cleavage of CUB domain-containing protein 1 (CDCP1). In some embodiments, the blocking includes occupying, by the composition, a lysine binding spot on plasminogen and blocking formation of plasmin. In some embodiments, the method includes modulating, by the composition, inflammatory and immune response in cancer patients via inhibition of plasmin to thereby enhance immune response.

In some embodiments, the synthetic lysine analog, derivative, or mimetic can include, without limitation, tranexamic acid, a combination of tranexamic acid and an additional positively charged amino acid, epsilon-aminocaproic acid (EACA), and AZD 6564, or substituted by a natural or synthetic arginine or histidine analog or derivative, or another amino acid derivative or analog. In some embodiments, the cancer can include, without limitation, colon cancer, blood cancers, lymphoma, leukemia, breast cancer, prostate cancer, stomach cancer, lung cancer, kidney cancer, pancreas cancer, skin cancer, neuroblastoma, glioma, glioblastoma, and combinations thereof. In some embodiments, the composition is in a solution. In some embodiments, the solution has a concentration of about 1 to about 60% by weight of the synthetic lysine analog, derivative, or mimetic. In some embodiments, the solution has a concentration of about 1 to about 30% by weight of the synthetic lysine analog, derivative, or mimetic. In some embodiments, the synthetic lysine analog, derivative, or mimetic is applied as part of a vehicle which adapts to human skin. In some embodiments, the solution containing the synthetic lysine analog, derivative, or mimetic is administered intravenously. In some embodiments, the solution is applied via a vehicle that allows the synthetic lysine analog, derivative, or mimetic to be delivered in a time-released fashion. In some embodiments, the composition is delivered orally. In some embodiments, the composition is delivered in a time-released fashion. In some embodiments, the administering of the composition occurs at least once a day.

In some embodiments, the composition is administered by at least one of a mechanical device, a permanent or resorbable material, and a vehicle whereby the synthetic lysine analog, derivative, or mimetic is delivered in a time-released fashion. In some embodiments, the composition is administered via a transdermal patch. In some embodiments, the composition is administered via an injected or implanted liposomal delivery depot. In some embodiments, the composition includes ingredients that provide for rapid systemic penetration or extended release. In some embodiments, the administering is performed systemically. In some embodiments, the composition is administered from about 1 mg/kg to about 100 mg/kg every 6-8 hours.

In some embodiments, the composition includes an additional therapeutic agent. In some embodiments, the additional therapeutic agent has a mechanism of action complimentary to the composition including the synthetic lysine analog, derivative, or mimetic.

In some embodiments, the synthetic lysine analog, derivative, or mimetic is at least one of a lysine prodrug, a lysine analog prodrug, a synthetic lysine analog prodrug, a lysine derivative prodrug, and a lysine mimetic prodrug. In some embodiments, the method further includes administering a co-therapy in conjunction with the composition. In some embodiments, the co-therapy results in synergistic benefits. In some embodiments, the co-therapy can include, without limitation, immunotherapy, gene therapy, chemotherapy, radiation, radiation therapy, electric fields, temperature therapies, surgical intervention, therapeutic treatments, ultrasonic therapy, ultrasound therapy, and combinations thereof. In some embodiments, the administering of the composition is conducted between multiple co-therapy treatments. In some embodiments, the administering of the composition is delivered separately or as a single therapy dose.

In an additional embodiment, the present disclosure relates to a method for prevention of cancer. In some embodiments, the method includes administering, prophylactically, a composition including a synthetic lysine analog, derivative, or mimetic. In some embodiments, the synthetic lysine analog, derivative, or mimetic interacts with potential cancer cells to inhibit or prevent formation of cancer cells.

In some embodiments, the method includes disrupting, by the composition, at least one of protein-protein, protein-DNA, and protein-RNA interaction by antagonizing at least one of lysine, arginine, and histidine residues on a protein. In some embodiments, the method includes blocking, by the composition, directly or indirectly acetylation or methylation of lysine or arginine residues. In some embodiments, the blocking results in acetylation inhibition of at least one of a p300 histone and a CREB-binding protein (CBP). In some embodiments, the method includes blocking formation of plasmin to thereby inhibit cleavage of CUB domain-containing protein 1 (CDCP1). In some embodiments, the blocking includes occupying, by the composition, a lysine binding spot on plasminogen and blocking formation of plasmin.

In some embodiments, the synthetic lysine analog, derivative, or mimetic is selected from the group consisting of tranexamic acid, a combination of tranexamic acid and an additional positively charged amino acid, epsilon-aminocaproic acid (EACA), and AZD 6564, or substituted by a natural or synthetic arginine or histidine analog or derivative, or another amino acid derivative or analog. In some embodiments, at least one of the potential cancer cells and the cancer cells are cells from a type of cancer that can include, but is not limited to, colon cancer, blood cancers, lymphoma, leukemia, breast cancer, prostate cancer, stomach cancer, lung cancer, kidney cancer, pancreas cancer, skin cancer, neuroblastoma, glioma, glioblastoma, and combinations thereof. In some embodiments, the composition is in a solution. In some embodiments, the solution has a concentration of about 1 to about 60% by weight of the synthetic lysine analog, derivative, or mimetic. In some embodiments, the solution has a concentration of about 1 to about 30% by weight of the synthetic lysine analog, derivative, or mimetic. In some embodiments, the synthetic lysine analog, derivative, or mimetic is applied as part of a vehicle which adapts to human skin. In some embodiments, the solution containing the synthetic lysine analog, derivative, or mimetic is administered intravenously. In some embodiments, the solution is applied via a vehicle that allows the synthetic lysine analog, derivative, or mimetic to be delivered in a time-released fashion. In some embodiments, the composition is delivered orally. In some embodiments, the composition is delivered in a time-released fashion. In some embodiments, the administering of the composition occurs at least once a day.

In some embodiments, the composition is administered by at least one of a mechanical device, a permanent or resorbable material, and a vehicle whereby the synthetic lysine analog, derivative, or mimetic is delivered in a time-released fashion. In some embodiments, the composition is administered via a transdermal patch. In some embodiments, the composition is administered via an injected or implanted liposomal delivery depot. In some embodiments, the composition includes ingredients that provide for rapid systemic penetration or extended release. In some embodiments, the administering is performed systemically. In some embodiments, the composition is administered from about 1 mg/kg to about 100 mg/kg every 6-8 hours.

In some embodiments, the composition includes an additional therapeutic agent. In some embodiments, the therapeutic agent has a mechanism of action complimentary to the composition including the synthetic lysine analog, derivative, or mimetic.

In some embodiments, the synthetic lysine analog, derivative, or mimetic is at least one of a lysine prodrug, a lysine analog prodrug, a synthetic lysine analog prodrug, a lysine derivative prodrug, and a lysine mimetic prodrug. In some embodiments, the method further includes administering a co-therapy in conjunction with the composition. In some embodiments, the co-therapy results in synergistic benefits. In some embodiments, the co-therapy can include, without limitation, immunotherapy, gene therapy, chemotherapy, radiation, radiation therapy, electric fields, temperature therapies, surgical intervention, therapeutic treatments, ultrasonic therapy, ultrasound therapy, and combinations thereof. In some embodiments, the administering of the composition is conducted between multiple co-therapy treatments. In some embodiments, the administering of the composition is delivered separately or as a single therapy dose.

In another aspect, the present disclosure relates to a composition for treatment or prevention of cancer. In some embodiments, the composition includes a synthetic lysine analog, derivative, or mimetic. In some embodiments, the synthetic lysine analog, derivative, or mimetic interacts with cancer cells to inhibit or prevent replication, survival, or formation of the cancer cells.

In some embodiments, the synthetic lysine analog, derivative, or mimetic disrupts at least one of protein-protein, protein-DNA, and protein-RNA interaction by antagonizing at least one of lysine, arginine, and histidine residues on a protein. In some embodiments, the synthetic lysine analog, derivative, or mimetic blocks directly or indirectly acetylation or methylation of lysine or arginine residues. In some embodiments, the blocking results in acetylation inhibition of at least one of a p300 histone and a CREB-binding protein (CBP). In some embodiments, the synthetic lysine analog, derivative, or mimetic blocks formation of plasmin to thereby inhibit cleavage of CUB domain-containing protein 1 (CDCP1). In some embodiments, the blocking includes the synthetic lysine analog, derivative, or mimetic occupying a lysine binding spot on plasminogen and blocking formation of plasmin. In some embodiments, the synthetic lysine analog, derivative, or mimetic modulates inflammatory and immune response in patients to thereby ameliorate the disease or disease condition.

In some embodiments, the synthetic lysine analog, derivative, or mimetic can include, without limitation, tranexamic acid, a combination of tranexamic acid and an additional positively charged amino acid, epsilon-aminocaproic acid (EACA), and AZD 6564, or substituted by a natural or synthetic arginine or histidine analog or derivative, or another amino acid derivative or analog. In some embodiments, the cancer can include, without limitation, colon cancer, blood cancers, lymphoma, leukemia, breast cancer, prostate cancer, stomach cancer, lung cancer, kidney cancer, pancreas cancer, skin cancer, neuroblastoma, glioma, glioblastoma, and combinations thereof. In some embodiments, the synthetic lysine analog, derivative, or mimetic is in a solution. In some embodiments, the solution has a concentration of about 1 to about 60% by weight of the synthetic lysine analog, derivative, or mimetic. In some embodiments, the solution has a concentration of about 1 to about 30% by weight of the synthetic lysine analog, derivative, or mimetic. In some embodiments, the synthetic lysine analog, derivative, or mimetic is applied as part of a vehicle which adapts to human skin. In some embodiments, the solution containing the synthetic lysine analog, derivative, or mimetic is administered intravenously. In some embodiments, the solution is applied via a vehicle that allows the synthetic lysine analog, derivative, or mimetic to be delivered in a time-released fashion.

In some embodiments, the synthetic lysine analog, derivative, or mimetic is delivered orally. In some embodiments, the synthetic lysine analog, derivative, or mimetic is delivered in a time-released fashion. In some embodiments, the synthetic lysine analog, derivative, or mimetic is administered at least once a day. In some embodiments, the synthetic lysine analog, derivative, or mimetic is administered by at least one of a mechanical device, a permanent or resorbable material, and a vehicle whereby the synthetic lysine analog, derivative, or mimetic is delivered in a time-released fashion. In some embodiments, the synthetic lysine analog, derivative, or mimetic is administered via a transdermal patch. In some embodiments, the synthetic lysine analog, derivative, or mimetic is administered via an injected or implanted liposomal delivery depot. In some embodiments, the synthetic lysine analog, derivative, or mimetic includes ingredients that provide for rapid systemic penetration or extended release.

In some embodiments, the synthetic lysine analog, derivative, or mimetic is systemically administered. In some embodiments, the synthetic lysine analog, derivative, or mimetic is administered from about 1 mg/kg to about 100 mg/kg every 6-8 hours. In some embodiments, the synthetic lysine analog, derivative, or mimetic includes an additional therapeutic agent. In some embodiments, the additional therapeutic agent has a mechanism of action complimentary to the synthetic lysine analog, derivative, or mimetic.

In some embodiments, the synthetic lysine analog, derivative, or mimetic is at least one of a lysine prodrug, a lysine analog prodrug, a synthetic lysine analog prodrug, a lysine derivative prodrug, and a lysine mimetic prodrug. In some embodiments, the synthetic lysine analog, derivative, or mimetic is administered in conjunction a co-therapy. In some embodiments, the co-therapy results in synergistic benefits. In some embodiments, the co-therapy can include, without limitation, immunotherapy, gene therapy, chemotherapy, radiation, radiation therapy, electric fields, temperature therapies, surgical intervention, therapeutic treatments, ultrasonic therapy, ultrasound therapy, and combinations thereof. In some embodiments, the synthetic lysine analog, derivative, or mimetic is administered between multiple co-therapy treatments. In some embodiments, the synthetic lysine analog, derivative, or mimetic is delivered separately or as a single therapy dose.

The term "substantially" is defined as largely but not necessarily wholly what is specified, as understood by a person of ordinary skill in the art. In any disclosed embodiment, the terms "substantially", "approximately", "generally", and "about" may be substituted with "within [a percentage] of" what is specified, where the percentage includes 0.1, 1, 5, and 10 percent.

The foregoing outlines features of several embodiments so that those skilled in the art may better understand the aspects of the disclosure. Those skilled in the art should appreciate that they may readily use the disclosure as a basis for designing or modifying other processes and structures for carrying out the same purposes and/or achieving the same advantages of the embodiments introduced herein. Those skilled in the art should also realize that such equivalent constructions do not depart from the spirit and scope of the disclosure, and that they may make various changes, substitutions, and alterations herein without departing from the spirit and scope of the disclosure. The scope of the invention should be determined only by the language of the claims that follow. The term "comprising" within the claims is intended to mean "including at least" such that the recited listing of elements in a claim are an open group. The terms "a", "an", and other singular terms are intended to include the plural forms thereof unless specifically excluded.

## Claims

1. A composition for ameliorating or preventing a disease or disease condition, for treatment or prevention of cancer, the composition comprising:
a synthetic lysine analog, derivative, or mimetic, wherein the synthetic lysine analog, derivative, or mimetic is the only therapeutic agent administered to the subject, wherein the synthetic lysine analog, derivative or mimetic is tranexamic acid, and wherein the composition is in a solution as part of a vehicle which adapts to human skin, and wherein the synthetic lysine analog, derivative, or mimetic interacts with cells associated with the cancer to inhibit or prevent replication, survival, or formation of the cells.

2. The composition of claim 1, wherein the synthetic lysine analog, derivative, or mimetic disrupts at least one of protein-protein, protein-DNA, and protein-RNA interaction by antagonizing at least one of lysine, arginine, and histidine residues on a protein or wherein the synthetic lysine analog, derivative, or mimetic modifies at least one of a lysine, arginine, and histidine residue of a protein by at least one of acetyl-CoA and acetyl-phosphate to thereby block a binding site on the protein or wherein the synthetic lysine analog, derivative, or mimetic occupies a lysine binding spot on plasminogen and blocking formation of plasmin or wherein the synthetic lysine analog, derivative, or mimetic modulates inflammatory and immune response in patients to thereby ameliorate the disease or disease condition.

3. The composition of claim 1, wherein the synthetic lysine analog, derivative, or mimetic blocks directly or indirectly at least one of acetylation, deacetylation, methylation, and demethylation processes of at least one of a lysine, arginine, or histidine residue of at least one of a histone or non-histone protein and optionally wherein the blocking results in acetylation inhibition of at least one of p300 histone acetyltransferase, a CREB-binding protein (CBP), and MYC.

4. The composition of claim 1, wherein the disease or disease condition is cause by an overexpression of at least one of p300 or MYC.

5. The composition of claim 1, wherein the compositions provide improvements in delivery, stimulates mitochondrial biogenesis, suppresses mitophagy, or stimulates mitochondrial respiration and optionally wherein the improvement in delivery is improved cellular penetration.

6. The composition of claim 1, wherein the synthetic lysine analog, derivative, or mimetic is in a solution and optionally wherein the solution is adapted to be delivered orally to accommodate greater concentrations of the synthetic lysine analog, derivative, or mimetic; or wherein the solution has a concentration of about 1 to about 60% by weight of the synthetic lysine analog, derivative, or mimetic; or wherein the solution has a concentration of about 1 to about 30% by weight of the synthetic lysine analog, derivative, or mimetic; or wherein the solution is applied as part of a vehicle which adapts to human skin, is applied via inhalation, is applied via nebulization methods, is applied via a spray, is applied via a nasal spray, and combinations thereof; or wherein the solution containing the synthetic lysine analog, derivative, or mimetic is administered intravenously; or wherein the solution is applied via a vehicle that allows the synthetic lysine analog, derivative, or mimetic to be delivered in a time-released fashion.

7. The composition of claim 6, wherein the solution is administered directly to an area of interest or administered to an area and transported to the area of interest and optionally wherein the area of interest is selected from the group consisting of an organ, a tumor, a diseased location, a malady location, the olfactory nerve and subsequently the brain, and combinations thereof.

8. The composition of claim 6, wherein the solution is delivered to an area via a percutaneous needle injection into an area of interest and optionally: wherein the area of interest is selected from the group consisting of an organ, a tumor, a diseased location, a malady location, and combinations thereof; or wherein a target location of the area of interest is monitored in real-time to deliver the percutaneous needle injection; or wherein the solution is applied via a vehicle that allows the synthetic lysine analog, derivative, or mimetic to be delivered in a time-released fashion.

9. The composition of claim 1, wherein the synthetic lysine analog, derivative, or mimetic is delivered orally, delivered via tablet or capsule, sublingually, via buccal delivery, or via mucosal delivery.

10. The composition of claim 1, wherein the synthetic lysine analog, derivative, or mimetic is administered from about 1 mg/kg to about 1000 mg/kg; or wherein the synthetic lysine analog, derivative, or mimetic is administered from about 20 mg/kg to about 200 mg/kg; or wherein the synthetic lysine analog, derivative, or mimetic is administered from about 100 mg/kg to about 1000 mg/kg.

11. The composition of claim 1, wherein the synthetic lysine analog, derivative, or mimetic is administered one time daily, two times daily, three times daily, four times daily, five times daily, or more or wherein the synthetic lysine analog, derivative, or mimetic is administered at least once a day, at least once every two days, at least once every three days, at least once every four days, at least once every five days, at least once every six days, at least once a week, at least once two weeks, at least once every three weeks, at least once every few weeks.

12. The composition of claim 1, wherein oral administration of the synthetic lysine analog, derivative, or mimetic comprises administration of a first tablet followed by a second tablet, and optionally wherein the second tablet is breakable in at least one location for continued administration.

13. The composition of claim 1, wherein the synthetic lysine analog, derivative, or mimetic is administered for a prescribed period of time, until the disease or disease condition is cured, continuously until end of life to treat, avoid or inhibit the disease or disease condition and optionally wherein the synthetic lysine analog, derivative, or mimetic is delivered in a time-released fashion or wherein the synthetic lysine analog, derivative, or mimetic is administered by at least one of a mechanical device, a permanent or resorbable material, and a vehicle whereby the synthetic lysine analog, derivative, or mimetic is delivered in a time-released fashion or wherein the synthetic lysine analog, derivative, or mimetic is administered via a transdermal patch or wherein the synthetic lysine analog, derivative, or mimetic is administered via an injected or implanted liposomal delivery depot.

14. The composition of claim 1, further comprising ingredients that provide for rapid systemic penetration or extended release.

15. The composition of claim 1, wherein the synthetic lysine analog, derivative, or mimetic is administered systemically, and optionally, wherein the synthetic lysine analog, derivative, or mimetic is administered from about 1 mg/kg to about 100 mg/kg every 6-8 hours.
